(19) Europäisches Patentamt
European Patent Office
Office européen des brevets

(11) EP 2 810 938 A1

(12) **EUROPEAN PATENT APPLICATION**
published in accordance with Art. 153(4) EPC

(43) Date of publication:
**10.12.2014 Bulletin 2014/50**

(21) Application number: **13733873.7**

(22) Date of filing: **04.01.2013**

(51) Int Cl.:
***C07D 219/00** (2006.01)* ***A61K 31/473** (2006.01)*
***A61P 35/00** (2006.01)*

(86) International application number:
**PCT/BR2013/000003**

(87) International publication number:
**WO 2013/102249 (11.07.2013 Gazette 2013/28)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **05.01.2012 BR 102012000187**

(71) Applicants:
- **Universidade Federal Do Rio De Janeiro - UFRJ 21941-901 Rio De Janeiro - RJ (BR)**
- **Uniao Brasiliense de Educacao e Cultura- UBEC 71.735-513 Nucleo Bandeirante (Brasilia - DF) (BR)**

(72) Inventors:
- **MARTINS DA SILVA, Claudia Lucia**
  **Rio de Janeiro - RJ (BR)**
- **CHAGAS DA SILVA, Fernanda**
  **CEP: 23013-620 Rio de Janeiro - RJ (BR)**
- **CHAVES BARBERATO, Luana**
  **CEP: 72115-115 Brasília - DF (BR)**
- **DE CAMARGO NASCENTE, Luciana**
  **CEP: 72110-800 Brasília - DF (BR)**
- **BARBOSA DO NASCIMENTO VIANA, Jessica**
  **CEP: 22210-060 Rio de Janeiro - RJ (BR)**
- **OLIVEIRA SILVA, Renata**
  **CEP: 72110-090 Barsília - DF (BR)**
- **NUNES LEMES, Laís Flávia**
  **CEP: 72210-086 Brasília - DF (BR)**
- **GERMAN NOËL, François**
  **CEP: 21940-380 Jardim Guanabara - RJ (BE)**
- **SOARES ROMEIRO,Luís Antônio**
  **CEP: 71906-500 Brasília - DF (BR)**

(74) Representative: **KATZAROV S.A.**
**European Patent Attorneys**
**19, rue des Epinettes**
**1227 Genève (CH)**

# (54) N-PHENYLPIPERAZINE DERIVATIVES THAT ARE ANTAGONISTS OF A1A, A1D ADRENOCEPTORS AND 5-HT1A RECEPTORS FOR THE TREATMENT OF BENIGN PROSTATE HYPERPLASIA, PHARMACEUTICAL COMPOSITIONS CONTAINING THE SAME

(57) The present invention provides N-phenylpiperazine derivatives for use as multiple binders and/or antagonists of $\alpha$1A adrenoceptors, $\alpha$1D adrenoceptors and 5-HT1A serotonin receptors. These substances are candidates to prototypes for the treatment of benign prostate hyperplasia and lower urinary tract symptoms, and are useful in pharmaceutical compositions.

EP 2 810 938 A1

## Description

### Specification of the Patent of Invention

N-phenylpiperazine derivatives that are antagonists of α1A, α1D adrenoceptors and 5-HT1A receptors for the treatment of benign prostate hyperplasia, pharmaceutical compositions containing the same.

### Field of the Invention

The present invention is in the technical field of pharmaceutical products and processes. More specifically, the present invention provides N-phenylpiperazine derivatives as ligands and / or multiple adrenoceptor of α1A adrenoceptors, α1D adrenoceptors and serotonin 5-HT1A receptors. Such substances are candidates for prototypes for treatment of benign prostatic hyperplasia and lower urinary tract symptoms and are useful in pharmaceutical compositions.

### Background of the Invention

*G-protein coupled receptors (GPCR)*

Receptors coupled to the heterotrimeric G protein have seven transmembrane domains and are also known as "G protein-coupled receptors" (GPCR) (Oldham & HA MM, Nature 9:60-71, 2008).
According to the *International Union of Basic and Clinical Pharmacology* (IUPHAR) - Receptor Database, in humans, these receptors are divided into classes, including Rhodopsin family (R) (Fredriksson et al Mol. Pharmacol. 63:1256-1272, 2003.).
According OVERINGTON and collaborators (Nat. Rev. Drug Discov. 5 (12) :993-996, 2006), drugs used clinically that have as pharmacological target this family of GPCRs correspond to approximately one quarter of the world pharmaceutical market. This is justified because the main role of these receptors is to recognize that a variety of endogenous and exogenous ligands extracellulars as hormones, proteins, lipids, neurotransmitters, among others (BJARNADOTTIR RN et al, Genomics 88:263-273, 2006: OLDHAM & HAMM, Nature 9:60-71, 2008), which involves them in various physiological and pathophysiological mechanisms. As an example, the activation of GPCR in the male urogenital system regulates from muscle contraction to cell proliferation and differentiation (AVELLAR et al., An. Acad. Bras. Cienc. 81 (3): 321-344, 2009). The ligands that act on these receptors show a remarkable degree of structural diversity (WESS Pharmacol. Ther. 80 (3) :231-264, 1998).
R family of GPCR's show great similarity in amino acid sequence in specific regions of these receptors, particularly in the hydrophobic transmembrane region of ligand recognition. This can be checked, for example, in the α1A adrenoceptors and 5-HT1A receptors, which have Asp residue in TM3 region committed to interaction with protonated nitrogen group of the ligand, and Ser and Tyr residues in the TM5 region important in the recognition of the ligand via hydrogen bonding. Considering these two receptors, these ligands recognition regions have 45% homology to each other (reviewed in OLDHAM & HAMM, Nature 9:60-71, 2008). Furthermore, there is a structural relationship between the ligands of these receptors that correspond with the nitrogen molecules still containing a protonatable groups aromatic region (FREDRIKSSON et al. Mol Pharmacol 63: 1256-1272, 2003).

*Adrenoceptors*

In humans, the α1A, α1B and α1D adrenoceptors are encoded by distinct genes (HIEBLE et al. Pharmacol Rev 45:267-270, 1995; MICHEL et al, Naunyn Schmiedebergs Arch Pharmacol 352 (1): 1 -10, 995), whose main structural feature consists in a high similarity degree in the amino acid sequence, showing similarity of about 65-73% in their domains (LANGER, Eur. J. Urol 36:. 2-6, 1999; VARMA & DENG, Can. J. Physiol. Pharmacol. 78:267-292, 2000; ZHONG & MINNEMAN, Eur. J. Pharmacol. 375:261-276, 1999; HUH et al., Genes Genet. Syst. 85 (l):65-73, 2010). Three subtypes have broad tissue distribution and may be a predominance of a particular subtype as showed in the mRNA study with rat tissues performed by SCOFIELD et al. (J. Pharmacol. Exp. Ther. 275: 1035-1042, 1995). In this study we can observe the predominance of the α1A subtype in the prostate and bladder, α1B in the liver, and α1D in the aorta of the rat.
In the autonomic nervous system, al α1 adrenoceptors are responsible for the modulation of non-vascular smooth muscle contraction and also vascular and therefore have major importance in the control of vascular tone and hence the control of blood pressure (HIEBLE, Pharm. Acta. Helv. 74 (2-3): 163-171, 2000; MICHELOTTI et al., Pharmacol. Ther. 88:281-309, 2000).
It is important to point out their actions the male urogenital system. Several studies have characterized the presence of these receptors in the lower urinary tract, especially in human prostatic tissue and bladder detrusor muscle

(FORRAY et al, Mol. Pharmacol. 45 (4):703-708, 1994; HATANO et al, Br. J. Pharmacol. 113 (3): 723-728, 1994; MARSHALL et al, J. Pharmacol. 115 (5): 781-786, 1995; HIEBLE & RUFFOLO, Expert Opin Investig Drugs. 6(4): 367-387, 1997; MALLOY et al, J. Urol. Sep; 160 (3 Pt 1) :937-943, 1998; MURAMATSU et al. Br J. Urol. 74 (5):572-578, 1994; HIEBLE et al., Pharmacol. Rev. 45:267-270, 1995; MICHELOTTI et al, Pharmacol. Ther. 88:281-309, 2000). In this system the α1 adrenoceptors are involved in the contraction of the bladder base, mainly via α1A adrenoceptor and α1D adrenoceptor, respectively (Chen et al., J. Urol. 174:370-374, 2005).

α1 adrenoceptor antagonists have been widely used for the treatment of benign prostatic hyperplasia (BPH), clinical condition that affects an increasing proportion of male population, causing obstruction of urinary flow due to an increase in the prostate size (MCNEAL, Urol. Clin. North Am., 17, 477-486, 1990). The prevalence of BPH increases with age, therefore, it jumps from about 50 % of men with 60 years age, reaching 80% of men with 80 age (COCKETT et al. Prog. Urol. 1 (6 ): 957-72, 1991).

It was found that the α1A subtype adrenoceptor is predominant in the human prostate at a level of RNAm (Price et al, Mol Pharmacol 46:221-226, 1994; TSENG-CRANK et al, J. Pharmacol. 115: 1475-1485, 1995) and of protein (LEPOR et al, J. Urol 149:640-642, 1993; MICHEL et al, J. Auton Pharmacol 16:21-28, 1996). Recent studies show that α1D subtype is also present in the prostate, at a significant extent (KOJIMA et al., Prostate 66:761-767, 2006). Thus, the use of drugs with higher affinity profile for α1A adrenoceptors and α1D adrenoceptors can be effective in the treatment of BPH and more tolerable than nonselective α1 adrenoceptor antagonist (CHAPPLE, Br J Urol 76 (1) :47-56, 1995), since it avoids adverse effects.

*α1 Adrenoceptor bindings*

Many chemical classes have the capacity to be recognized by α1 adrenoceptors. Among the drugs with high affinity for the three α1A, α1B and α1D receptor subtypes, it is found the prazosin antagonist (SHIBATA et al., Mol. Pharmacol. 48:250-258, 1995), quinazoline derivative used in the treatment of hypertension.

Another important chemical class includes phenylpiperazine derivatives such as BMY 7378, α1D adrenoceptor antagonist with affinity in the nanomolar range and used as a pharmacological tool (GOETZ et al, Eur. J. Pharmacol. 272 (2-3): R5-6, 1995). Furthermore, BMY 7378 is a partial agonist of serotonin 5-HT1A receptor (CHAPUT & MONTIGNI J. Pharmacol. Ther. 246 (1): 359-370, 1988).

*Serotonergic Receptors (5-HT)*

In mammals, it is known seven families of 5-HT receptors (5-HT1-7), for a total of 14 structurally and pharmacologically distinct subtypes of 5-HT receptors ((HOYER et al, Pharmacol Rev 46:157-203, 1994; HOYER & MARTIN, Neuropharmacology 36:419-428, 1997; SAXENA et al, Trends Pharmacol Sci 19:311-316, 1998; VILLALON et al, Drug Discov Today 2:294-300, 1997; VILLALON & CENTURION, Naunyn. Schmiedebergs Arch Pharmacol 376 (1-2) :45-63 2007). Considering the primary structure, the 5-HT metabotropic receptors have a high degree of similarity to adrenergic and dopaminergic receptors (FREDRIKSSON et al. Mol Pharmacol.:1256-1272 63, 2003).

Metabotropic subtypes 5-HT1A and 5-HT2A show greater similarity with α1 adrenoceptor receptors, in about 45% (TRUMPP-KALLMEYER et al. J. Med. 35(19):3448-3462. 1992) and moderate homology (35%) between them (BARNES & SHARP Neuropharmacology 38:1083-1152, 1999).

*5-HT1A Receptors*

The 5-HT1A receptor was one of the first receptors of this family which have the gene cloned (KOBILKA et al Nature 329:75-79, 1987; Fargin et al Nature 335 (6188):358-360, 1988). RNAm studies of the receptor 5-HT1A show the expression in the brain, which are present in high density in the cerebral cortex. However, they are also found outside the CNS, such as the spleen, kidney (neonatal), intestine (PUCADYIL & CHATTOPADHYAY, Progr. Lipid Res. 45:295-333, 2006) and prostate (ABDUL et al. Anticancer Res 14 (3A): 1215-1220, 1994; DIZEYI et al Prostate 59 (3) :328-336, 2004).

It is known that prostate neuroendocrine cells synthesize, store and release growth factors and neuropeptides including 5-HT (ABRAHAMSSON et al. 1986), having specific receptors for 5-HT in these tissues (HOOSEIN et al. 1993). Thus, the potential use of 5-HT1A antagonists has been highlighted for the treatment of BPH since they have an anti-proliferative effect in tumor prostate cells and are useful in controlling the growth of androgen independent tumors (ABDUL and cols. Anticancer Res 14 (3A):1215-1220, 1994; DIZEYI et al Prostate 59 (3) :328-336, 2004). The chemical structures shown below are examples of some of the chemical classes which have affinity for 5-HT1A (*structures*

*taken from IUPHAR Receptor Database):*

*Hypothetical model of molecular recognition*

The determination of the crystal structure of GPCRs in general is hampered by difficult to reproduce in models of crystallization of natural conformation of these receptors that have seven transmembrane domains. For example, only recently, the first crystallography of a GPCR has been obtained, in case the human β2-adrenoceptor and subsequently A2a adenosine receptor (reviewed in TATE and SCHERTLER, Curr Opin Struct Biol 19 (4): 386 - 395 2009;. KOBILKA and SCHERTLER, Trends Pharmacol Sci 29 (2) :79-83, 2008). Therefore, the construction of predictive models and the study of interactions with known ligands are important for obtaining new knowledge about these important pharmacological targets (THIEL, Nat Biotechnol 22:513-519, 2004).

Mutagenesis studies has indicated that the protonated nitrogen of the α adrenoceptors ligands, such as phenylpiperazine derivatives, carry our ionic interactions with a aspartate residue in the TM3, the amino group in the form of positive ion, which is considered as main pharmacophore group (PEREZ, Biochem Pharmacol 73 (8): 1051 -1062, 2007). On the other hand, the aromatic rings carry out hydrophobic and electrostatic interactions with complementary residues in TM2, 4, 6 and 7 (WAUGH et al J. Biol Chem 276 (27): 25366-2537, 2001). In addition to these important attachments, the presence of an aromatic ring containing acceptors groups of hydrogen bond in the positions *meta* and *para* are relevant in the stabilization of the linker-receptor complex to interact with Ser residues in TM5 (LOPEZ-RODRIGUEZ et al. J. Med. Chem 40, 2653-2656, 1997; PEREZ Biochem Pharmacol 73 (8): 1051-1062, 2007).

The α1 adrenoceptor and the 5-HT1A receptors show a great similarity in ligand recognition transmembrane region (TRUMPP-KALLMEYER et al. J. Med. 35 (19) : 3448-3462, 1992). There has been evidence that an important site for binding of α1A-adrenoceptor antagonists and 5-HT1A receptors is a Asp residue in the TM3, which interacts with the protonated amino group of the ligands (LI et al. J. Mol. Model 14 (10) :957-966,2008; LOPEZ-RODRIGUEZ et al Mol Pharmacol 62 (1): 15-21, 2002). Several strategies are available for the molecular design of drugs. Among these the most important is the physiological approach, which is based on the mechanism of pharmacological action desired (KUBINYI. Pharmazie 50; 647-662 1995), which depends on selection of the therapeutic target, which in turn depends on knowledge of the involved pathophysiological process. Chronic diseases may involve alteration in the cellular signing of more than one type of receptor, and therefore drugs that are targeted to act on different receptors may be therapeutically useful (HUBER et al Biochemistry 47 (42): 11013-1 1023, 2008).

Several studies have shown the presence of α1 adrenoceptors within the urinary tract, particularly in human prostate tissue and bladder detrusor, which have their increased expression in BPH (FORRAY et al. Pharmacol. 45 (4) :703-708, 1994 ; HATANO et al J. Pharmacol 113 (3) :723-728, 1994; Marshall et al Br J Pharmacol 115 (5): 1-786 78, 1995; HIEBLE & RUFFOLO Expert Opin Investig Drugs 6 (4) :367-387, 1997;. MALLOY et al Urol, J. et al Sep. 160 (3 Pt 1) :937-943,1998; MURAMATSU et al Br J Urol. 74 (5) :572-578, 1994; HIEBLE et al Pharmacol Rev. 45:267-270, 1995; HIEBLE Pharma Helv Acta 74 (2-3): 163-171, 2000; MTCHELOTTI et al. Pharmacol. Ther. 88:281-309, 2000). In addition to these receptors, 5-HT1A receptors are also expressed in human prostate tissue where they exert a proliferative effect (ABDUL et al, Anticancer Res 14 (3A): 1215-1220, 1994; DIZEYL et al Prostate 59 (3) :328-336, 2004). In view of this, there is great interest in the field of development of new drugs for dual antagonists for the treatment of BPH since it has an interest in the block of α1A/D and 5-HT1A adrenoceptors receptors.

A search of the scientific literature showed no relevant document related to N-phenylpiperazine derivatives as multiple antagonists of α1A adrenoceptors, α1D adrenoceptor and 5-HT1A serotonergics as new candidates to prototypes for the treatment of benign prostatic hyperplasia and lower urinary tract symptoms described in the present invention.

Then it appears form the studied literature that no documents were found suggesting anticipating or the teachings of the present invention, so the solution proposed herein has novelty and inventive step in face of the prior art.

**Summary d Invention**

Objects of the present invention are N-phenylpiperazine derivatives, ligands, pharmaceutical compositions containing them.

N-phenylpiperazine derivatives of the invention are particularly useful as binders *for in vitro* processes and are also particularly useful in pharmaceutical compositions for the treatment of benign prostatic hyperplasia and / or lower urinary tract symptoms, in addition to anti-proliferative effect, including the cell proliferation of androgen-independent tumor prostatic origin. Considering the α1 adrenoceptors and 5-HT1A receptors, the N- phenylpiperazine the invention have structural prerequisites necessary for binding to these receptors, such as basic nitrogen atom for ionic interaction with the carboxylate group of the aspartate residue (Asp) in TM3 of these receptors (adrenoceptors α1 and 5-HT1A receptors), in addition to the aromatic ring for hydrophobic and electrostatic interactions with complementary residues in TM2, 4, 6 and 7.

Another object of the invention is the process of obtaining the N- phenylpiperazine which have variation in alkyl

chain.

These and other objects of the invention will be immediately appreciated by those skilled in the art and by companies with interests in the segment, and it will be described in sufficient details to reproduce the description below.

**Brief Description of the Figures**

Figure 1 shows concentration-response curves to phenylephrine (PE) in rat aorta in the absence (■) or presence (□) of the vehicle used for dilution of LDT's. Data are expressed as mean ± EPM (n = 5).

**Detailed Description of the Invention**

The present invention provides N-phenylpiperazine derivatives, ligands, pharmaceutical compositions containing them.

N-phenylpiperazine derivatives of the invention are particularly useful as binders for in vitro processes and are also particularly useful in pharmaceutical compositions for the treatment of benign prostatic hyperplasia and / or lower urinary tract symptoms, in addition to anti-proliferative effect, including the cell proliferation of androgen-independent tumor prostatic origin. In particular, a 2-alcoxyphenylpiperazine derivative of the invention have high affinity for α1A and α1D adrenoceptors and 5-HT1A receptors and comprises:

- A basic nitrogen atom for ionic interaction with the carboxylate group of the aspartate residue (Asp) in TM3 of these receptors; and
- At least one aromatic ring for hydrophobic and electrostatic interactions with complementary residues in TM2 4, 6 and 7; and
- A group with a negative charge density at position 2 (orto) of the nitrogen or the N-phenylpiperazine subunit, hydrogen bond acceptor e.g. alkoxides and isosteres.

Several compounds were synthesized with N-phenylpiperazine pharmacophore grouping. The chemical structures of the compounds of the list LDT 2-6, LDT8, LDT 62-69, LDT 39, 70 and LDT 245 are listed below:

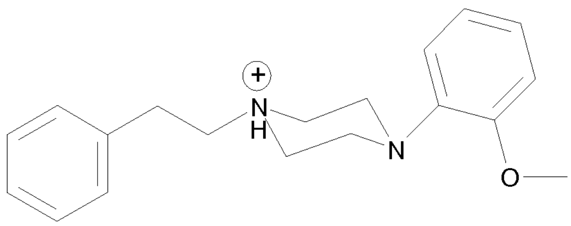

LDT 2

1-(2-phenylethyl)-4-(2-methoxyphenyl)piperazine

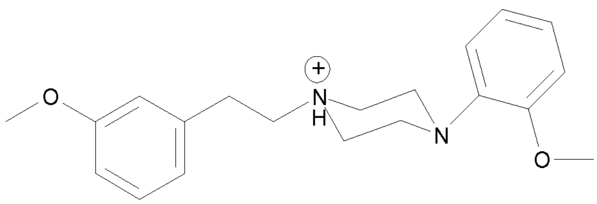

LDT 3

1-(2-methoxyphenyl)-4-[2-(3-methoxyphenyl)ethyl] piperazine

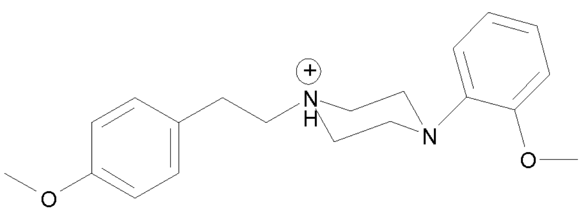

LDT 4

1-(2-methoxyphenyl)-4-[2-(4-methoxyphenyl)ethyl] piperazine

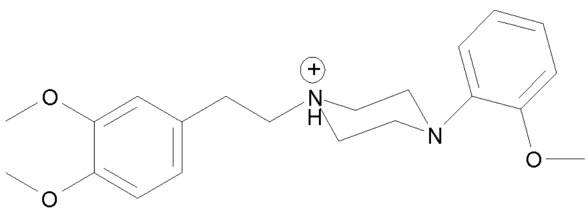

LDT 5

1-(2-methoxyphenyl)-4-[2-(3,4-dimethoxyphenyl)ethyl] piperazine

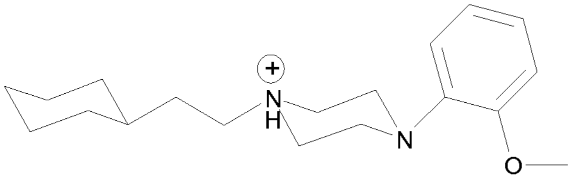

LDT 6

1-(2-cyclohexylethyl)-4-(2-methoxyphenyl) piperazine

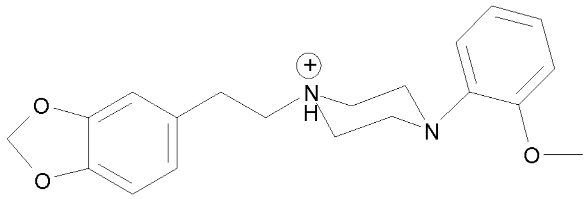

LDT 8

1-(1,3-benzodioxol-5-ilethyl) -4-(2-ethoxyphenyl)piperazine

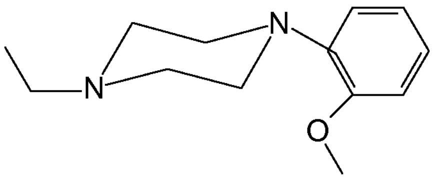

LDT 62

1-ethyl-4-(2-methoxyphenyl) piperazine

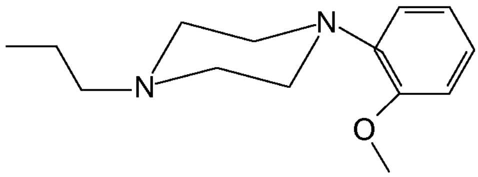

LDT 63

1-(2-methoxyphenyl)-4-propylpiperazine

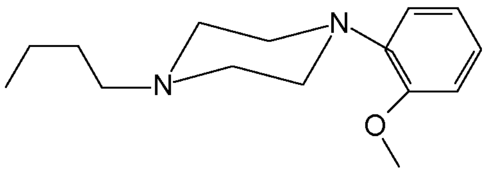

LDT 64

1-butyl-4-(2-methoxyphenyl) piperazine

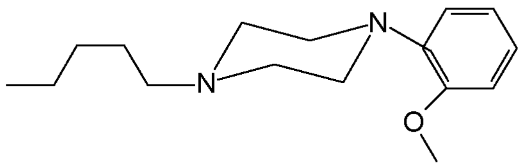

LDT 65

1-(2-methoxyphenyl)-4- pentyl piperazine

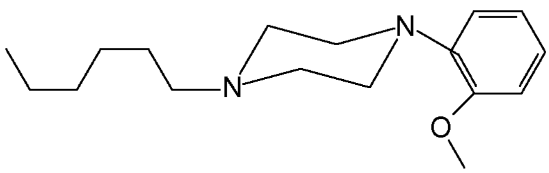

LDT 66

1-hexyl-4-(2-methoxyphenyl) piperazine

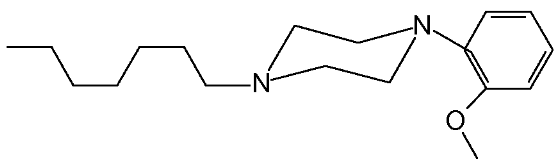

LDT 67

1-heptyl-4-(2-methoxyphenyl) piperazine

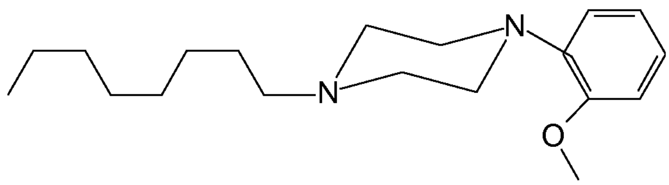

LDT 68

1- (2-methoxyphenyl)-4-octylpiperazine

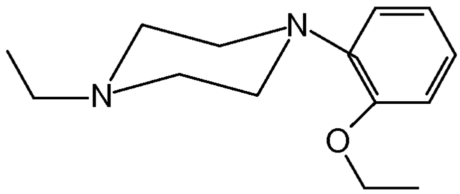

LDT 69

1-ethyl-4-(2-ethoxyphenyl) piperazine

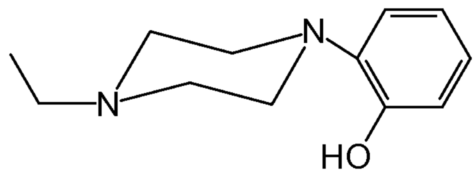

LDT 70

1-ethyl-4-(2-hydroxyphenyl) piperazine

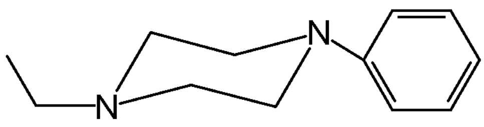

LDT 39

1- ethyl-4-(2-hydroxyphenyl)piperazine

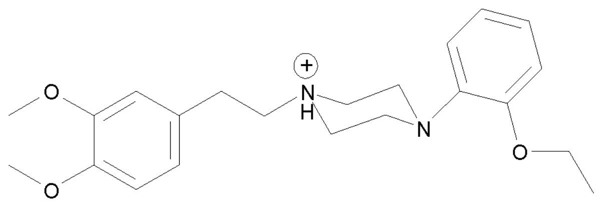

LDT 245

1 (-2-ethoxyphenyl)-4-[2-(3,4-dimethoxy)ethyl]piperazine

There are two studies with N-phenylpiperazine derivatives for α1 adrenoceptors and 5-HT1A receptors, one of Ratouis et al (J. Med. 8:271-273, 1965), and the other of Leopold et al (J Pharm. Pharmacol. 56 (2) :247-255, 2003) who studied, among others, the phenylpiperazines sometimes called LDT5 in the first, and LDT3, LDT4 LDT5 in the second study. In addition, there are also two other references characterizing LDT62-68 as binders for 5-HT1A and 5-HT2 (MOKROSZ et al, Arch Pharm 328: 143-148, 1995), and also a reference for LDT66 and LDT68 as just α1 adrenoceptor ligands (MOKROSZ et al, J Med Chem 39: 1125-1129, 1996). However, none of these references presents determination of the intrinsic activity, and systematic analysis, in parallel, of the reasons for selectivity for the 5-HT2A receptor and α1 and α2 adrenoceptors. All LDT2-6-LDT8 and LDT62-68 is presented as a series of potential candidates in the context of the search for antagonists with dual affinity for al-adrenoceptors receptors α1 A / D and 5-HT1A.

*Obtaining the novel N-phenylpiperazine*

All derivatives called in the present invention as LDT's were synthesized from the corresponding alkyl halide, commercial or previously synthesized from the corresponding primary alcohols, using SN2 reaction with N-phenylpiperazines in acetonitrile in the presence of amine base or carbonate at reflux in a conventional or microwave heating, in yields ranging from 86 to 97%, and made available in the form of monohydrochloride with subsequent solubilization at a concentration of 10 mM in ultrapure water and storage at -20 ° C.

Infra-red spectra were registered by Fourier transform (FT-IR) on a Perkin Elmer spectrometer (Spectrum BX) spectrum IH-NMR (300 and 500 MHz CDC13) and 13 C-NMR (75 and 125 MHz CDC13) those were recorded in more Varian Plus (7.05 T) and Bruker Avance DRX300 and DRX500 spectrometer, and mass spectra were recorded on

spectrometer Shimadzu LCMS IT-TOF. The plates of the thin layer chromatography (TLC) eluted with a mixture of polar and nonpolar solvents (ethanol / chloroform) showed a single spot indicating the presence of only one compound in each sample, as confirmed by spectrometric analysis. Every day of the experiment, small aliquots of the storage solution in concentrations necessary to perform the experiment in question were diluted.

All experiments using LDT2-6, LDT8 and LDT62-68 and LTD-45 series were performed under light pressure sodium lamp in order to avoid possible degradation of the compounds.

*Organs obtaining*

All protocols were previously approved by the ethics committee of UFRJ (CEUA; protocol: DFBCICB011).

Male Wistar rats from 2.5 to 3 months were euthanized by decapitation after being anesthetized with ether in saturated chamber. Male albino rabbits (2.5 to 3 kg) were euthanized by exsanguination after being anesthetized with intravenous pentobarbital (40 mg / kg).

*Radioligands and Drugs*

The radioligands [3H]-prazosin (specific activity 85 Ci / mmol), [3H] - ketanserin (specific activity 67 Ci / mmol), [3H]-8-OH-DPAT (specific activity 170.2 Ci / mmol), [3H] mPPF, [3H]-QNB, [3H]-YM09151-2 and [3H] RX821002 were purchased from PerkinElmer, USA, and [3H]RX821002 (specific activity 60 Ci / mmol) was purchased from Amersham, UK. Prazosine hydrochloride, bitartrate adrenaline, pargilina hydrochloride, 5-hydroxytryptamine hydrochloride, acetylcholine hydrochloride, (R) - (- )-phenylephrine hydrochloride, (±)-propranolol hydrochloride, and BMY7378 dihydrochloride and ketanserina tartrate were purchased from SIGMA, USA.

*Functional Assays*

Isometric contraction assays were performed using Male Wistar rats, aged 2.5 - 3 months euthanized as described above. The thoracic aorta (tissue enriched in functional α1D adrenergic receptors) was removed, free from adjacent connective and adipose tissues cut in 3 mm segments. Then, each ring was fixed to a tension transducer (Grass FT-03) and immersed in vats containing 9 mL of saline (NaCl 122 mM, KCl 5 mM, CaCl2 1.25 mM, MgCl2 1.25 mM, KH2PO 1,25 mM, NaHCO3 15 mM, glucose 11.5 mM) maintained at 37 ° C under constant aeration with carbonic mixture (95% 02 and 5% CO2). The aort segments were then subjected to a preload of 20 mN per 60 minutes. After recovery of tissues maintained for 1 hour at rest, it was performed a contraction induced by phenylephrine (PE) 1 μM (α1 adrenergic receptors selective agonist). In this plateau contraction, in order to promote endothelium-dependent relaxation resulting from the activation of the enzyme endothelial nitric oxide synthase (eNOS) and subsequent release of nitric oxide (NO), acetylcholine 1 μM was added. Rings that relaxed at least 80% were considered to have intact endothelium, were used.

Then the preparation was washed and allowed to stand for 1 hour in order to recover the tissue. Then it was performed cumulative curves to phenylephrine ($10^{-9}$ to $10^{-5}$ M) in the presence of propranolol 1 μM (β-adrenoceptor blocker receptors) before and after incubation for 1 hour of the LDT's at 10 or 50 nM. In parallel, a temporal control was performed to rule out any artifacts on the measured contraction, where was added only the vehicle (ultrapure water Milli Q ®) instead of the substance to be tested. Strictly following the same protocol as the other tanks wherein it was added the LDT's.

The data were scanned in MacLab 8S system connected to the isometric tension transducer Grass FT-03, which recorded the voltage variation in milinewtons (mN) generated throughout the analysis. The digitized data were then analyzed by Chart 3.4 / s software program (MacLab, Inc., USA). In the pharmacological screening process, the initial concentration of the used LDT's was 50 nM, which is considered as cut concentration, i.e., substances that have no effect at this concentration would not investigated in this test. In contrast, the substances that were in effect, were then tested at lower concentrations (10 or 2 nM) (Table 2). Thus, by constructing concentration-response curves to the agonist PE in the absence and presence of the antagonist, it can be determined the maximum effect parameters (Emax) and the concentration which promotes Emax of 50% (EC50). The ratio of the EC50 value in these two conditions (with and without LDT) matches the third parameter, called "concentration ratio" (or RC). Once it has determined these parameters it is possible to calculate the apparent affinity of the competitive antagonist (KB) *in vitro* using the Schild equation (Eq. 1) (KENAKIN, Raven Press, New York. Pp :278-322, 1993).

$$\text{(Equation 1)} \log (CR-1) = \log [B] - \log KB$$

Where CR = concentration ratio (EC'50 / EC50); [B] = 'B' antagonist concentration; KB = equilibrium dissociation constant of the antagonist 'B'.

*Data analysis and statistical treatment*

The tension data were analyzed by non-linear regression to calculate the maximum effect parameters (Emax) and concentration which promotes Emax 50% (EC50) using GraphPad Prism software, version 4 (U.S.). To determine whether there was significant difference between the measurements taken before and after treatment with LDT's it was conducted single-factor ANOVA test followed by Newman-Keuls test, considering a 5% alpha risk (differences were considered significant if $P < 0.05$).

*Functional Tests with prostate*

Isometric contraction assays were performed using Male Wistar rats, aged 2,5 - 3 months euthanized as described above. The male rat ventral prostate was removed and dissected into 10 mm segments. Then, each segment was fixed to a tension transducer (GRASS FT-03) and immersed in vats containing 9 ml of saline solution maintained at 37 ° C under constant aeration with carbon mixture ( 95% 02 and 5% CO2). The segments were then subjected to a preload of 10 mN per 60 minutes. 1 μM Phenylephrine-induced contraction (PE) (α1 adrenergic selective agonist) was performed. Then the preparation was washed and allowed to stand for 1 hour in order to recover the tissue. It was carried out cumulative curves to phenylephrine ($10^{-8}$ - $10^{-3}$ M) in the presence of 1 μM propranolol (β-adrenoceptor blocker receptors) before and after incubation for 1 hour of the LDT's 3, 10 or 100 50 nM. In parallel, a time control was performed.

Data were digitized at MacLab 8S system connected to the isometric tension transducer Grass FT-03, which recorded the voltage variation in milinewtons (mN) generated throughout the analysis. The digitized data were then analyzed using Chart 3.4 / s software program (MacLab, Inc., USA). Thus, by constructing concentration-response curves to the agonist phenylephrine in the absence and presence of the antagonist it can be determined the parameters maximum effect (Emax) and the concentration that promotes Emax of 50% (EC50). The ratio of the EC50 value in these two conditions (with and without LDT) is the third parameter called "concentration ratio" (or CR). After this parameters were determined, it was possible to calculate the apparent affinity of the competitive antagonist (KB) *in vitro* using the Schild equation (KENAKIN, Raven Press, New York. Pp :278-322, 1993).

*Statistical Analysis*

To determine whether there was significant difference between the measured values it was performed an ANOVA single factor test followed by Newman-Keuls test, considering an alpha risk of 5% (differences were considered significant if $P < 0.05$).

*Binding assays* (*Binding*)

*α1A adrenoceptors*

*Membrane Preparation*

Following the protocol described previously in the literature, membrane preparations from rabbit liver homogenates were obtained. Such membrane preparations are enriched in adrenoceptor subtype α1A. The tissue obtained as described above (~ 30 g) was naturally thawed, immersed in thawing solution (0.25 M sucrose, 1 mM EGTA and 5 mM Tris (pH 7.4)) freezing. Then, the tissue was transferred to a petri dish on ice containing solution for homogenizing liver (2 mM EDTA, 100 mM NaCl and 50 mM Tris (pH 7.4)) freezing. The liver was then carefully cut by ignoring the most fibrous part.

Then, the material was transferred into a beaker where with the aid of Ultra-Turrax homogenizer (speed 24,000 RPM), the tissue was homogenized with the same buffer solution at a ratio of 6 parts of the solution to 1 part material. The material was homogenized three times for 20 seconds; in the intervals the solution was allowed to stand for 1 minute on ice. Subsequently, the homogenate was subjected to centrifugation 10,000 x g for 10 minutes at 4 ° C, yielding a supernatant which was subjected to ultracentrifugation 80,000 x g for 40 minutes at 4 ° C yielding a pellet. This pellet was resuspended in buffer solution without NaCl (1 mM EDTA and 50 mM Tris (pH 7.4)) and further subjected to ultracentrifugation in the same previous conditions. The pellet obtained was resuspended in this step storage solution (0.25 M sucrose, 1 mM EGTA and 5 mM Tris (pH 7.4)) with the aid of a manual type Dounce homogenizer and stored in aliquots at 300 μL liquid nitrogen. In all protocols for membrane preparations the dosage of protein were performed according to the method of LOWRY et al. (J. Biol. Chem. 193 (1) :265-275, 1951).

Binding of [3H]-prazosina to α1A adrenoceptors

The standardization of the assay was done as described in the literature. In the saturation test it was measured the binding of [3H]-prazosin to native α1A -adrenoceptor the absence and presence of different concentrations of non-radioactive prazosin ($10^{-10}$ - $10^{-7}$ M). For competition assays, test tubes containing 350 μL of intermediate solution ([3H]-prazosin 0.16 nM, 1.6 mM EDTA, 50 mM Tris (pH 7.4 at 25°C)) it was added the 50 μL of different concentrations of LDT's (end concentration $10^{-9}$ - $10^{-4}$ M) or 50 μL of water (solvent for dilution of LDT's) for the determination of total binding or 50 μL nonradioactive prazosin at 10 μM (end 1 μM) to the determination of nonspecific binding, and 200 μg of membrane preparations of rabbit liver (α1A) contained in 50 μL suspension completing end volume of 500 μL. Assays were performed in triplicate.

In both assays, membrane preparations were incubated at 0° C for 45 minutes. After the incubation period, the reaction was stopped by adding 4 mL of ice-cold wash buffer (50 mM Tris-HCl, pH 7.4) followed by rapid vacuum filtration on glass fiber filter (GMF 3 Filtrak ®). The filters were washed four times with 4 ml of the same solution under vacuum to remove all free radioligand. Then the filters were dried and placed in vials containing 5 ml scintillation fluid (4% PPO, 0.1% POPOP w / v in toluene). The radioactivity retained on the filters was then determined in a liquid scintillation counter (Packard Tri-Carb 1600 TR).

Specific binding of [$^3$H]-prazosin to α1A adrenoceptors was defined as the difference between total binding and non-specific binding. Data obtained by saturation experiment allowed us to obtain the parameters Kd (dissociation constant), which is the concentration of radioligand required to occupy 50% of the receptor sites and Bmax, maximum density of binding sites, which were calculated by non-linear regression. The increased values of bound were calculated indirectly by the value obtained for the concentration of bound radioligand (fmol / mg protein) which was multiplied by the dilution factor (DF) of radioligand (e.g. [3H]-prazosin) results from adding the nonradioactive ligand (e.g., prazosin), in a process known as isotope dilution. This procedure aims to obtain a saturation curve with less expenditure of radioactive substances and biological materials. Since competition assays allow us to construct inhibition curves of specific binding of radioligand. The higher the concentration of competitor agent employed in this case the LDT 's study, the greater the competition for specific receptors, decreasing the formation radioligand-receptor complex. This can be seen by the gradual decrease of radioactivity retained on the filters is detected by the counter liquid scintillation.

From these curves it was possible to calculate the LDT's concentration values which inhibits 50% radioligand binding (IC50).These values were converted to Ki values (competitive dissociation constant at equilibrium or inhibition constant) using the Cheng-Prusoff equation (Eq. 2).

$$\text{(Equation 2)} \quad Ki = \frac{CI_{50}}{1 + ([L] / Kd)}$$

Where Ki = inhibition constant; CI50 = concentration inhibiting 50% radioligand binding; [L] = concentration of radioligand; Kd = equilibrium dissociation constant of the ligand (measured in saturation assay).

*α1B adrenoceptors,*

*Membrane Preparation*

Following the protocol described previously in the literature membrane preparations from rat liver homogenates were obtained. Such membrane preparations are enriched in the adrenoceptor subtype α1B. The tissues obtained as described above (4 Rat livers corresponding to ~30 g) were thawed and homogenized perforated similarly performed for rabbit liver. The homogenate obtained was filtered through four layers of gauze and then subjected to centrifugation 5,000 x g for 20 minutes at 4 ° C, yielding a supernatant which was subsequently ultracentrifuged at 100,000 x g for 60 minutes at 4 ° C, obtaining a pellet. This pellet was resuspended in buffer free of NaCl and subjected to a further ultrafiltration under the same conditions. The pellet obtained in this step was resuspended in storage solution (0.25M sucrose, 1mM EGTA TIIM and 5 mM Tris (pH 7.4)) with the aid of a manual type Dounce homogenizer and stored in aliquots of 300 μL in liquid nitrogen.

*Binding of [3H]-prazosin to α1B adrenoceptors*

Binding assays were standardized for α1B adrenoceptors in the same way adrenoceptor binding assays for α1A according to the literature, i.e. saturation and competition assays were performed on membrane preparations from rat liver, in this case using 150 g protein per tube contained 50 MI suspension, in addition to various concentrations of

LDT's ($10^{-9}$ - $10^{-6}$ M).

*$\alpha$2 adrenoceptors and muscarinic receptors*

*Membrane Preparation*

Membrane preparations of rat cortex as described previously in the literature were performed. The organs were homogenized in a Potter with the aid of a motor unit (Fisatom) and Teflon piston in the proportion of 20 parts of 50 mM Tris HCl (pH 7.4 at 4 ° C) for one piece of material, three times for 30 seconds each time, in the intervals solution was rest on ice for 1 minute.

Then it was performed a centrifugation at 900 x g for 10 minutes at 4 ° C, which was obtained a pellet which was resuspended in ice-cold 50 mM Tris-HCl solution (pH 7.4) and the same procedure was performed once more. The supernatant was then centrifuged at 48,000 x g for 10 minutes at 4 ° C yielding a pellet which was resuspended at a ratio of 20 parts of 50 mM Tris HCl (pH 7.4 at 4° C) for 1 part material, and incubated at 37 ° C for 10 minutes to remove endogenous neurotransmitters. Finally, centrifugation two times at 48,000 x g was performed for 10 minutes at 4 ° C, and then the final pellet was resuspended in a solution of 50 mM Tris-HCl (pH 7.4).

*$\alpha$2 and 5-HT2A adrenoceptors receptors*

*Membrane Preparation*

Membrane preparations of rat cortex as described previously in the literature were performed. The organs were homogenized in a Potter with the aid of a motor unit (Fisatom) and Teflon piston in the proportion of 20 parts of 50 mM Tris HCl (pH 7.4 at 4 ° C) for one piece of material, three times for 30 seconds each time, at intervals the solution was allowed to stand on ice for 1 minute.

Then it was performed a centrifugation at 900 x g for 10 minutes at 4 ° C. It was obtained a pellet which was resuspended in solution cold 50 mM Tris-HCl (pH 7.4) and the same procedure was performed once more. The supernatant was then centrifuged at 48,000 x g for 10 minutes at 4 ° C yielding a pellet which was resuspended at a ratio of 20 parts of 50 mM Tris HCl (pH 7.4 at 4 ° C) for one piece of material, and incubated at 37 ° C for 10 minutes in order to remove the endogenous neurotransmitters. Finally, centrifugation two times more at 48,000 x g was performed for 10 minutes at 4 ° C, and then the final pellet was resuspended in a solution of 50 mM Tris-HCl (pH 7.4).

*Binding of the [3H]RX821002 to $\alpha$2 adrenoceptors*

Binding assays for the $\alpha$2A receptor were performed as described previously in the literature and previously standardized in the laboratory by saturation experiments. The values of Kd and Bmax were 2.05 $\pm$ 0.28 nM and 124 $\pm$ 7 fmol / mg protein (n = 1), respectively. Incubate for 60 minutes at 30 ° C, 150 membrane preparation of rat cortex contained 50 $\mu$L was suspended in 50 mM Tris-HCl into tubes containing 400 $\mu$L intermediate solution ([3H] RX821002 1.25 nM, 50 mM Tris (pH 7.4 at 37 ° C) and 50 $\mu$L of different concentrations of LDT's (final concentration $10^{-7}$ - 3 x$10^{-5}$ M to LDT65-68) or 50 $\mu$L of 50 mM Tris-HCl pH 7.4 at 37 ° C (solvent for dilution of the LDT's), to determine the total binding or 50 $\mu$L nonradioactive bitartrate adrenaline at 100 $\mu$M for determining the non-specific bounding (final volume 500 $\mu$L). Assays were performed in triplicate.

After the incubation period, the reaction was stopped by adding 4 mL ice cold solution containing 5 mM Tris (pH 7.4), followed by vacuum filtration on glass fiber filter (GMF 3, Filtrak ®) previously soaked with solution 0.5% polyethylenoimine. The filters were washed three times with 4 mL of ice-cold solution containing 5 mM Tris (pH 7.4) under vacuum to remove all free radioligand. Then the filters were dried and placed in vials containing 5 ml scintillation fluid (composition: 4% PPO, 0.1% POPOP w/v in toluene). The radioactivity retained on the filters was then determined in a liquid scintillation counter (Packard Tri-Carb 1600 TR). Specific binding of [3H]RX821002 to $\alpha$2A receptors was defined as the difference between total binding and non-specific binding. To these receptors were determined and IC50 and Ki parameters.

*Binding of [$^3$H]-QNB to muscarinic receptors*

Binding assays for muscarinic receptors were performed as described in the literature (RICHARDS J Pharmacol 99, 753-761, 1990; CASTOLDI and cols., Life Sci. 78: 1915-1924, 2006). 150 g membrane preparations of rat cortex were incubated with 0, 1 radioligand nM [$^3$ H]-QNB, in medium containing 50 mM Tris-HCl (pH 7.4 at 4 ° C) in the absence and presence of LTD's derivative (0.1 - 100 $\mu$M) for 60 min at 25 ° C. Non-specific binding was determined in the presence of 1 $\mu$M atropine sulfate. Assays were performed in triplicate. The radioactivity retained on the filters was then determined

in a liquid scintillation counter (Packard Tri-Carb 1600 TR). To these receptors were determined the IC50 and Ki parameters.

*5-HT1A receptors*

*Membrane Preparation*

Membrane preparations of rat hippocampus according to previously described by HALL et al (1985) and PEROUTKA (1986) were performed. The homogenization and centrifugation steps were performed identically to that described above for receptors $\alpha$2A and 5-HT2A receptors.

*Binding of [3H]-8-OH-DPAT or [3H]p-MPPF to 5-HT1A receptors*

The binding assays for the 5-HT1A receptors were performed as described previously in the literature and previously standardized in the laboratory through saturation experiments. The values of Kd and Bmax were 0.7 $\pm$ 0.1 nM and 125 $\pm$ 42 fmol / mg protein, respectively (Neves et al., Bioorg Med. Chem. 18 (5): 1925-1935, 2010). It was incubate for 15 minutes at 37 ° C, 50 mg of membrane preparation of rat hippocampus, 50 $\mu$L contained in suspension in 50 mM Tris-HCl, into tubes containing 400 $\mu$l intermediate solution ([3H]-8-OH-DPAT 1.25 mM, 1.25 mM CaC12, 1.25 mM MnC12, 10 $\mu$M pargyline (in order to protect the possible degradation of the radioligand by the monoamine oxidase enzyme (MAO), 50 mM Tris (pH 7, 4, 37 ° C) addition of 50 $\mu$L different concentrations of LDT's (final concentration $10^{-10}$ - $10^{-6}$ M to LDT 2-6; $10^{-12}$ - $10^{-6}$ M to LDT 8, and $10^{-9}$ - $10^{-4}$ M to LTD 62-70 and LDT 39) or 50 $\mu$L of 50 mM Tris-HCl pH 7.4 at 37 ° C (solvent for dilution of LDT's) for the determination of total binding, or 50 $\mu$L non-radioative serotonin at 10 $\mu$M to determine the non-specific binding (final volume of 500 $\mu$L). Assays were performed in triplicate. Alternatively, incubated for 45 minutes at 37 ° C, 50 $\mu$g of membrane preparation of rat hippocampus contained in 50 $\mu$L suspension in 50 mM Tris-HCl, into tubes containing 400 Ml intermediate solution ([3H]p-MPPF 0.625 nM, 1.25 GTP, and 50 mM Tris (pH 7.4 at 37 ° C) in addition to 50 $\mu$L of different concentrations of LTD's (final concentration $10^{-9}$ - 3 x $10^{-7}$ M to 65-68 LDT) or 50 $\mu$L, Tris- HCl 50 mM pH 7.4 at 37 ° C (solvent for dilution of LDT's) for the determination of total binding, and in the same manner as in binding of [3 H] 8-OH-DPAT, 50 $\mu$L nonradioactive serotonin at 10 $\mu$M for the determination of non-specific binding (final volume of 500 $\mu$L). Assays were performed in triplicate.

After the incubation period, the reaction was stopped by adding 4 ml of ice-cold solution containing 5 mM Tris (pH 7.4), followed by vacuum filtration on glass fiber filter (GMF 3, Filtrak ®) previously soaked with solution 0.5% polyethylenoimine. The filters were washed three times with 4 mL of ice-cold solution containing 5 mM Tris (pH 7.4) under vacuum to remove all free radioligand. Then the filters were dried and placed in vials containing 5 ml scintillation fluid (composition: 4% PPO, 0.1% POPOP w/p in toluene). The radioactivity retained on the filters was then determined in a liquid scintillation counter (Packard Tri-Carb 1600 TR). Specific binding of [3H]-8-OH-DPAT for 5-HT1A receptors was defined as the difference between total binding and non-specific binding. For these receptors were also determined the LC50 and Ki parameters.

*Intrinsic activity at native* 5-HT1A *receptors*

The GTP presence displaces the GPCR to a state of low affinity (Lahti et al, 1992). The intrinsic activity of a substance can be estimated using GPCR agonist or antagonist radioligand appropriate to define the affinity of the substance in the state of high and low affinity receptor, respectively. Therefore, to determine the intrinsic activity of the more potent derivatives in 5HT1A receptors it was calculated their dissociation constants (Ki) using an antagonist as radioligand ([3H] p-MPPF) in the presence of a high concentration of GTP (Ki low) or an agonist ([3H] 8-OH-DPAT) in the absence of GTP (Ki high). Values for Ki ratio (Low Ki / High Ki) considerably greater than 1 (one) indicates agonism, whereas values close to 1 indicate antagonism while negative values indicate inverse agonism.

*[3H]-ketanserina binding to 5-HT2A receptors*

The binding assays for 5-HT2A receptors were performed as described in the literature. The values of Kd and Bmax obtained in previous laboratory experiments were 1.77 $\pm$ 0.07 nM and 348 $\pm$ 51 fmol / mg protein, respectively. Incubate for 15 minutes at 37 ° C, 150 $\mu$g of membrane preparation contained 50 $\mu$L of rat cortex suspension into tubes containing 400 $\mu$L intermediate solution ([3H]-ketanserin 1.25 nM, 125 nM prazosin , 50 mM Tris, HCl IN up to pH 7.4 at 37 ° C) in addition to 50 $\mu$L of different concentrations of LDT's (final concentration $10^{-10}$ - $10^{-4}$ M to LDT 2-4; and $10^{-8}$ - $10^{-4}$ to LDT 5-8) or 50 $\mu$L 50 mM Tris-HCl pH 7.4 at 37 ° C (solvent for dilution of the LDT's), to determine the total binding, or 50 $\mu$L non-radioactive ketanserin at 10 $\mu$M for the determination of nonspecific binding (final volume of 500 $\mu$L). Assays were performed in triplicate.

After the incubation period, the reaction was stopped by adding 4 ml of ice-cold solution containing 5 mM Tris (pH 7.4), followed by vacuum filtration on glass fiber filter (GMF 3, Filtrak ®) previously soaked with solution 0.5% polyethylenoimine. The filters were washed three times with 4 mL of ice-cold solution containing 5 mM Tris (pH 7.4) under vacuum to remove all free radioligand. Then the filters were dried and placed in vials containing 5 ml scintillation fluid (4% PPO, 0.1% POPOP w / v in toluene). The radioactivity retained on the filters was then determined in a liquid scintillation counter (Packard Tri-Carb 1600 TR). Specific binding of [3H]-ketanserin to the 5-HT2A receptor was defined as the difference between total binding and non-specific binding. To these receptors were also determined CI50 and Ki parameters.

*D2-Like Receptors*

*Membrane Preparation*

The membrane preparation of rat striatum (receptor $D_2$ - "like" - subtypes of dopamine receptors: $D_2$ , D3 and $D_4$) was performed according to literature description (Niznik et al, Naunyn-Schmiedebergs Arch.. Pharmacol 329:333-343, 1985; Terai et al. Eur J Pharmacol 173:177, 1989; HAMDI et al, Life Sci 50, 1529-1534, 1992). Briefly, organs were homogenized for 30 seconds three times in Fisatom in 50 mM Tris-HCl, $MgCl_2$ 8 mM, 5 mM EDTA (pH 7.4 at 4 ° C) and then centrifuged at 48,000 x $g_{av}$ (20 minutes 4 ° C). The pellet was resuspended in 50 mM Tris-HCl, 8 mM $MgCl_2$, 5 mM EDTA (pH 7.4 at 37 ° C), incubated at 37 ° C for 10 minutes and centrifuged twice more. The final pellet was resuspended in the same buffer at a concentration of 1.5 mL / g tissue.

*$D_2$-Like receptors binding assay*

$D_2$-Like receptors binding assays were performed as described by our group (Neves et al., Bioorg. Med. 18 (5), 1925-1935, 2010). 50 μg rat striatal membranes were incubated for 60 min at 37 ° C in medium containing 0.1 nM of the antagonist radioligand [$^3$H]-YM 09151-2, 120 mM NaCl, 5 mm KCl, 5 mM MgC12, 1.5 mM CaC12, 1 mM EDTA and 50 mM Tris-HCl (pH 7.2 at 25 ° C) in the presence or absence of LTD's (0,003-30 μM). Non-specific binding was measured in the presence of 30 μM sulpiride. Assays were performed in triplicate.

The radioactivity retained on the filters was then determined in a liquid scintillation counter (Packard Tri-Carb 1600 TR). For these receptors were also determined IC50 and Ki parameters.

*Protein dosage*

The dosage of the protein content of the samples was performed by the literature and proposed modified for microtiter plate (96 well plate) colorimetric method. To construct a standard curve used the protein bovine serum albumin (BSA) at concentrations of 50, 100, 200, 250, 350 μg / mL. It was added 50 μL patterns of protein or 50 μL of dilute protein samples under study to each well containing 250 μL of a 2% disodium carbonate in 0.1 N NaOH, 1% cupric sulphate and 2 % sodium potassium tartrate, 50 μL water (white). Finally, it was added 15 μL the Folin to each well and mixing with multichannel pipette. The plate was incubated for 45 min at room temperature from the homogenization of the first well. The absorbance values were obtained in plate spectrophotometer at a wavelength equal to 700 nm. The experiments were performed in triplicate using two different dilution factors. The calculation of the protein content of the samples was performed by interpolation using the values of the standard curve of absorbance versus protein concentration, which were analyzed by linear regression using the GraphPad Prism 4.0 program. Values are expressed in mg protein / ml homogenate.

*Data analysis and statistical treatment*

The data obtained in binding assays were analyzed by nonlinear regression using the GraphPad Prism software (USA) in order to calculate the parameters of the competition curves, IC50, and the saturation curve, such as Kd and Bmax. Specially for the saturation experiments in the α1A and α1B adrenergic receptors one or two binding sites were tested, by choosing the one that best applies through the F test (DE LEAN et al., Mol Pharmacol. 21 (1) :5 it, 1982). Thus, when the sum of squares of errors was greatly reduced by assuming the model of the two opposite sites of a site model, the first model was used to obtain estimates of Kd values of prazosin. On the other hand, when no improvement was significant when adding a second site, it was obtained a single Kd value.

The difference between experimental groups was analyzed by single factor analysis of variance (one way ANOVA) followed by post hoc test of Newman-Keuls (more than 2 groups) considering in both cases $P < 0.05$.

*Cell proliferation in vitro test*

Human prostate cancer cells (DU-145) were cultured in medium (RPMI 1640) until confluence. Then the cells were dissociated, plated (5 x $10^3$ cells / well) and maintained in medium free of fetal bovine serum for 24 hours. Then the cells were maintained and treated with 5-HT (1 $\mu$M) or phenylephrine (3 $\mu$M) in the absence or presence of LTD's (5, 50 or 500 nM) for 48 h. Cell proliferation was assessed by using the MTT technique (MOSMANN, T. Immunol Methods. 16;65 (1 -2) :55-63, 1983).

*Acute toxicity test*

Swiss mice (female, 25-30 g, 6 animals per condition) were treated with LDT via intraperitoneal with LTD's at a dose of 100 $\mu$g / kg and observed for 14 days in the behavioral framework according to described method (LORKE, D., Arch Toxicol, 54 (4):. 275 - 87, 1983, modified). The temperature was measured by retal probe before and after 30 and 60 min of described treatment.

*Phenylephrine-induced contraction inhibition in rat aorta*

According SCOFIELD et al (J. Pharmacol Exp Ther 275:. 1035-1042, 1995) as well as TESTA et al (Life Sci 57 (13). P1159-63, 1995), the rat thoracic aorta corresponds to a tissue expressing mainly $\alpha$1D-adrenoceptors in addition are enriched in serotoninergic receptors subtype 5-HT2A (Banes et al, J. Pharmacol Exp Ther 291:.. 1179-1187, 1999). With this in view, the functional tests of contraction in isolated organ model were performed using these tissues. When added to the incubation bath, none of the LDT's (50 mM) changed the baseline of isometric tension. Thus, was discarded a possible ruled-agonist effect on receptors with vasoconstrictor action, such as $\alpha$1-adrenoceptors and serotonin (5-HT2A), on this concentration. Similarly, targeting to rule out any artifacts on the measured contraction, a time control was performed where only the vehicle was added (ultrapure Mili Q® water) in place of the substance to be tested, strictly following the same protocol as the other vats were added to the LDT ' s (Fig. 1).

We conducted experiments with BMY7378 50 nM, $\alpha$1D adrenoceptor antagonist used as positive control, and obtained a value of KB = 2.95 nM, consistent with the literature (CARROLL et al, Bioorg Med Chem Lett, 11:1119 - 1121, 2001). Within the series of LDT's 2-6 and 8, all substances, except LDT6, reduced the contraction induced by FE, also inducing a shift of concentration-response curves to FE to the right, quantified by the ratio of CE50 (CR). Thus, the data suggest an $\alpha$1D adrenergic antagonist effect for all derivatives studied, except LDT6.Thereafter the apparent affinity of antagonists was calculated (Table 1). The second experimental condition (LDT's 10 nM) was conducted only for the LDT 5 and 8 or 2 nM (LDT 245), to determine the affinity (KB) calculated from the Schild equation. The entire series LDT 2-8 except LDT6, showed great affinity for $\alpha$1D adrenoceptors. Compared with BMY7378 (KB=2.95 nM) affinity for this receptor is greater for the LDT derivative 5 and 8 (KB = 0.57 and 0.16, respectively, * P <0.05).

Regarding other derivatives LDT's 62-70 and 39, all concentration-response curves to phenylephrine suffered a classic right shift, quantified by the ratio of EC50 (CR) (Table 4), in the presence of LDT's relative the control curve, thus behaving as competitive antagonists. Among the antagonists N-phenyl piperazine varying the alkyl chain attached to position R1 (LDT ' s 63-68), and comparing them with the LDT62 derivative, there was a significant increase in relative affinity (KB) by $\alpha$1D-adrenergic receptors for LDT66 and LDT67 derivatives(Table 2), in which the alkyl chain ranging from six to seven carbons, respectively, indicating that hydrophobic interactions are important for molecular recognition in this subtype of adrenergic receptor, which is consistent with the literature which describes the important role of hydrophobic forces in determining the affinity of N-phenylpiperazines or orto- methoxyphenyl piperazines for the $\alpha$ adrenoceptors.

The increase in hydrophobicity on LDT69, given by the substitution of R2 by an ethoxylated group did not alter the affinity to $\alpha$1D-adrenergic receptor relative to LDT62, wherein R2 is a methoxyl. Moreover, the inclusion of hydrophilic group donor and acceptor of hydrogen bond, or the substituent in R2 absence observed for LDT70 and LDT39, respectively, reduce affinity by $\alpha$1D - adrenergic receptors. The range of affinity of LDT's 66-68 for $\alpha$1D adrenoceptor is is equivalent to the affinity reported for other antagonists such as N-phenylpiperazine BMY7378 and naftopidil.

Table 1: Affinity of the LDT's (2-6, 8, 245) and BMY7378 by the $\alpha$1D adrenoceptors

| **LDT** | Log CE50 $\pm$ **EPM** (M) | | **CR$\pm$EPM** | *log KB* $\pm$ **EPM** (M) | *KB* (nM) | **N** |
|---|---|---|---|---|---|---|
| | Control | LDT | | | | |
| 2 a | -7,00 $\pm$ 0,19 | -5,65 $\pm$ 0,19 | 24,3 $\pm$ 8,7 | -8,51 $\pm$ 0,37 | 2,15 | 5 |
| 3 a | -6,87 $\pm$ 0,25 | -5,40 $\pm$ 0,25 | 29,1 $\pm$ 1,8 | -8,75 $\pm$ 0,03 | 1,78 | 5 |

(continued)

| LDT | Log CE50 ± **EPM** (M) | | **CR±EPM** | *log KB* ± **EPM** (M) | *KB* (nM) | **N** |
|---|---|---|---|---|---|---|
| | Control | LDT | | | | |
| 4 a | -6,99 ± 0,19 | -5,79 ± 0,32 | 16,9 ± 5,5 | -8,47 ± 0,16 | 3,14 | 5 |
| 5 b | -7,12 ± 0,22 | -5,85 ± 0,15 | 18,5 ± 2,5 | -9,23 ± 0,06 * | 0,57 | 6 |
| 6 a | ND | ND | ND | ND | ND | 7 |
| 8 b | -7,14 ± 0,10 | -5,35 ± 0,14 | 62,5±15,5 | -9,78 ± 0,19 * | 0,16 | 10 |
| 245 c | -6,72 ± 0,09 | -6,23 ± 0,14 | 3,9 ± 1,0 | -8,93 ± 0,19 | 1,16 | 7 |
| BMY7378b | -6,99 ± 0,15 | -6,36 ± 0,09 | 4,4 ± 0,8 | -8,53 ± 0,11 | 2,95 | 6 |
| KB values individually calculated and obtained from the functional experiments in the presence of LDT OR BMY7378 at 50 nM, b10 nM or c2 nM. The CR (ratio between the EC50 in the presence and absence LDTs) is the average of CRs calculated from EC50 obtained in the non-linear regression analysis. *P < 0.05 compared to BMY7378 (single factor ANOVA followed by Newman-Keuls test). | | | | | | |

Table 2. Affinity of the LTD's (62-70 and 39) by α1D adrenoceptors

| LDT | Log CE50 ± **EPM** (M) | | CR | *log KB* ± **EPM** (M) | *KB* (nM) | **N** |
|---|---|---|---|---|---|---|
| | Control | LDT | | | | |
| 62 | -6,86 ± 0,07 | -6,01 ± 0,06 | 7 | -8,08 ± 0,02 | 8,3 | 4 |
| 63 | -6,34 ± 0,14 | -6,80 ± 0,18 | 3,1 | -7,59 ± 0,12b | 31,5 | 5 |
| 64 | -6,91 ± 0,05 | -6,18 ± 0,08 | 6,4 | -7,93 ± 0,13 | 11,7 | 6 |
| 65 | -6,73 ± 0,02 | -5,89 ± 0,05 | 7,0 | -8,08 ± 0,04 | 8,3 | 4 |
| 66 | -6,78 ± 0,08 | -5,29 ± 0,07 | 32 | -8,78 ± 0,06a | 1,66 | 4 |
| 67 | -6,91 ± 0,11 | -5,46 ± 0,08 | 29,5 | -8,74 ± 0,08a | 1,82 | 4 |
| 68 | -6,91 ± 0,04 | -5,81 ± 0,06 | 12,8 | -8,36 ± 0,05 | 4,36 | 5 |
| 69 | -6,70 ± 0,08 | -5,87 ± 0,07 | 6,9 | -8,06 ± 0,08 | 8,71 | 3 |
| 70 | -6,93 ± 0,03 | -4,86 ± 0,09 | 123,6 | -7,07 ± 0,08a | 85,1 | 4 |
| 39 | -6,77 ± 0,08 | -4,91 ± 0,05 | 76,7 | -6,85 ± 0,08a | 141 | 5 |

KB values individually calculated and obtained from the functional tests in the presence of LTD 50 nM (LTD's 62-69) or 10 μM (LTD's 70 and 39). The CR (ratio between EC50 in the presence and absence of LTDs) is the medium of the CR's calculated from EC50 obtained by non-linear regression analysis. aP < 0,001 and bP < 0,01 compared to LTD62 (single factor ANOVA followed by Newman-Keuls test).

*Binding assays (Binding)*

Adrenoceptor α1A and α1B - Percentage of binding of [3H]-prazosin

First, saturation assays preparation of rabbit liver and rat were performed to determine the binding parameters of [3H]-prazosin. To adrenoceptor α1A after analysis considering the binding of the radioligand to one or two specific binding sites, the best curve fit was obtained considering the two-site model. In this case it has a high affinity site corresponding to the α1L type, and a second low affinity site, the α1L, whose existence, nature and function are discussed in the literature. While for α1B adrenoceptor at the best fit occurred in only one binding site model. By using prazosin non-radioactive as competitor agent to standardized curve competition for binding of 0.1 nM [3H]-prazosin. Accordingly, the competition curve was biphasic in preparation of rabbit liver, approximately 2/3 of the sites labeled by 0.1 nM [3H]-prazosin showing high affinity for prazosin (CI50 = 0.16 nM) and 1 / 3 had low affinity sites (CI50 1 nM).

Kd values of [3H]-prazosin to α1A adrenoceptors were 0.46 nM and 56.5 nM for sites with high and low affinities,

respectively (n = 3), and α1B adrenoceptors the Kd value was 0,34 nM (n = 3), in agreement with literature (SHIBATA and cols., Mol. Pharmacol., 48:250-258, 1995). Concerning the LDTs, all series tested was able to inhibit specific bounding of the [3 H]-prazosin in competition assays.

For the calculation of the Ki of the LTD's it was used the Cheng-Prusoff equation, where for prazosin (PZS) Kd values were 0.46 nM and 56.5 nM for sites of high and low affinity, respectively to α1A adrenoceptor, and it was used Kd = 0.34 nM for α1B-adrenoceptor. The obtained Ki values are summarized in the following Tables 3 to 5 below.

Table 3: pharmacological parameters of the LDT's (2-6 and 8) in the α1A-adrenoceptors

| LDT | | *log CI50* ± **EPM** *(M)* a | *Ki (M) b* | |
|---|---|---|---|---|
| | n | High Affinity site | High Affinity site | |
| 2 | 4 | -7,72 ± 0,26 | 1,57 x $10^{-8}$ | |
| 3 | 4 | -8,25 ± 0,67 | 4,63 x $10^{-9}$ | |
| 4 | 3 | -7,97 ± 0,79 | 8,81 x $10^{-9}$ | |
| 5 | 3 | -8,35 ± 0,20 | 3,67 x $10^{-9}$ | |
| 6 | 3 | -8,58 ± 0,34 | 2,16 x $10^{-9}$ | |
| 8 | 4 | -8,34 ± 0,71 | 3,76 x $10^{-9}$ | |
| PZS | 3 | -9,53 ± 0,15 | 2,43 x $10^{-10}$ | |

a CI50 values were calculated by non-linear regression using the GraphPad Prism software (USA) and expressed as mean ± EPM;
b Ki values were calculated using the Cheng-Prusoff equation;
P <0.05 compared to the PZS (single-factor ANOVA followed by Newman-Keuls).

Table 4: Pharmacological Parameters of the LDT's (2-6, 8) α1B adrenoceptor

| LDT | n | *log CI50* ± **EPM** *(M)* a | *Ki (M) b* |
|---|---|---|---|
| 2 | 4 | -7,20 ± 0,49 | 4,87 x $10^{-8}$ |
| 3 | 4 | -7,02 ± 0,31 | 7,37 x $10^{-8}$ |
| 4 | 4 | -7,52 ± 0,32 | 2,33 x $10^{-8}$ |
| 5 | 5 | -7,80 ± 0,37 | 1,22 x $10^{-8}$ |
| 6 | 3 | -6,70 ± 0,19 | 1,54 x $10^{-7}$ |
| 8 | 5 | -7,93 ± 0,39 | 9,07 x $10^{-9}$ |

a CI50 values were calculated by non-linear regression using the GraphPad Prism software (USA) and expressed as mean ± EPM;
b Ki values were calculated using the Cheng-Prusoff equation;

Table 5: Pharmacological parameters of LDT' s (62-70 and 39) in α1A and α1B adrenoceptor

| LDT | α 1A *(n)* | | α 1B *(n)* | |
|---|---|---|---|---|
| | log IC50 (M) | *Ki* (nM) | log IC50 (M) | *Ki* (nM) |
| 62 | -6,51 ± 0,2 | 261 (4) | -6,27 ± 0,05 | 448 (3) |
| 63 | -6,50 ± 0,08 | 270 (4) | -6,22 ± 0,05 | 511 (3) |
| 64 | -6,84 ± 0,04 | 124 (3) | -6,33 ± 0,16 | 393 (3) |
| 65 | -7,10 ± 0,06 | 67 (3) | -6,48 ± 0,16 | 277 (3) |
| 66 | -7,29 ± 0,11 a | 43 (4) | -6,51 ± 0,18 | 221 (4) |
| 67 | -7,02 ± 0,12 a | 81 (3) | -6,87 ± 0,10 | 113 (3) |

(continued)

| LDT | α 1A *(n)* | | α 1B *(n)* | |
|---|---|---|---|---|
| | log IC50 (M) | *Ki* (nM) | log IC50 (M) | *Ki* (nM) |
| 68 | -7,35 ± 0,2 a | 38 (3) | -6,69 ± 0,10 | 173 (3) |
| 69 | -7,41 ± 0,25 a | 33 (3) | -6,53 ± 0,11 | 185 (3) |
| 70 | -6,45 ± 0,07 | 304 (3) | -6,07 ± 0,10 | 715 (3) |
| 39 | -6,36 ± 0,21 | 374 (3) | -6,29 ± 1,10 | 425 (3) |
| a CI50 values were calculated by non-linear regression using the GraphPad Prism software (USA) and expressed as mean ± EPM;<br>b Ki values were calculated using the Cheng-Prusoff equation;<br>P <0.01 compared to the LTD62 (single-factor ANOVA followed by Newman-Keuls). | | | | |

All LDT 2-8 group showed besides the ability to identify two binding sites, Ki values in the nanomolar range to the high affinity component (α1A) similar to that observed for prazosin (Table 3). There was an increase in the affinity of the derivatives LDT65 to LDT69 compared to the LDT62 to the α1A adrenoceptors. LDT66, LDT68 and LDT69 derivatives (Ki equal to 43, 38 and 33 nM, respectively) showed affinity six to eight times higher (P <0.01) than LDT62 (Ki = 261 nM) (Table 5). Although LDT65 and LDT67 have Ki values from three to four times smaller (67 and 81 nM, respectively) compared to LDT62 this increase in affinity was not significant (P = 0.06 (LDT65) and 0.1 (LDT67)). There was no difference between affinity and LDT62 and LDT63 derivatives. LDT64, LDT39 and LDT70. For adrenoceptor α1B, once again there was a lower affinity profile for LDT6 in the range μM when compared to the others, especially the LDT8, nanomolar range, but the difference was not significant. Thus, we see that the auxophoric substitution observed in LDT6 does not contribute to the upregulation of the apparent affinity for α1 B / D adrenoceptor subtypes.

The LDT's 62-70 and LDT39 derivatives have lower affinity for α1B-adrenoceptors in relation to the α1A and α1D receptors. Although none of the structural changes has significantly altered the affinity, there seems to be an increased affinity related to the increase of the alkyl chain from LDT65 derivative. Moreover, there also seems to have greater interaction when the ethoxylated group in the R2 phenylpiperazine subunit position present in LDT69 (also observed for LDT8) compared with the LDT62 methoxyl, using the same standard affinity receptors α1A, but to a lesser degree. Mokrosz et al. (1997) found that only LDT's 66 and 68 are ligands of α1-adrenergic receptor, however, it was not determined their affinity. Thus, we evaluate by what α1 -adrenoceptor subtypes the LDT's would bind specifically and what would be its intrinsic activity in these subtypes. As for α1D adrenoceptors it seems that hydrophobic interactions with alkyl chain good size are important requirements for molecular recognition of a receptor α1A because the affinities (Table 6) were significantly higher for LDT66 and LDT68 derivatives, which have alkyl chain with 6 and 8 carbons, respectively.

The LTD's with size increase of the alkyl chain, more specifically LDT's 65-68 derivatives, LDT 66 and 68 are the most potent, are within the affinity range shown for most drugs in clinical use, which makes them new potential dual α1 adrenoceptor antagonists (α1A and α1d) which may be useful not only in relieving the symptoms of BPH but also to inhibit the proliferation of the prostatic tissue via α1A adrenoceptor antagonism.

*A2A receptors - binding percentage of [3H] RX821002*

Previously performed by our group, saturation assays in rat cortex, where it was determined the binding parameters of [3H] RX821002 (Kd = 2.05 ± 0.28 nM and Bmax = 124 ± 7 fmol protein / mg) to α2A receptors. This Kd value was used to calculate Ki of LDT's using Cheng-Prusoff equation.

During competition assays, the LDT's 65-68 were able to inhibit the specific binding of [3H] RX821002 to α2A adrenergic receptors, but with lower affinities (μM range, Table 5), as well as the α-1B adrenergic with respect to α1A / 1D adrenergic receptors. The fact that these substances present a low affinity for this receptor is important for there is a high homology between α2A adrenoceptor subtypes. Thus, the tested LDT's are likely to present little blocking of this α2A adrenoceptor that is involved with the regulation of central nervous, cardiovascular and male genitourinary systems, by inhibiting the release of catecholamines in these systems.

Table 6: Pharmacological Parameters of the LDT's (65-68) in α2A receptors.

| LDT | α 2A | |
|---|---|---|
| | log IC50 (M) | *Ki* (μM) (n) |
| 65 | -5,60 ± 0,06 | 1,5(5) |
| 66 | -5,92 ± 0,06 | 0,8(5) |
| 67 | -6,10 ± 0,06 | 0,5(5) |
| 68 | -6,01 ± 0,03 | 0,6(5) |

IC50 values were calculated by non-linear regression using GraphPad Prism software (USA) and expressed as the mean ± EPM of (n) individual experiments; Ki values were calculated using the Cheng-Prusoff equation.

The 5-HT1A receptors - binding percentage of [3H]-8-OH-DPAT

Saturation studies in rat hippocampal enriched tissue in 5-HT1A receptors were performed to determine the binding parameters of the [3H]-8-OH-DPAT (Kd = 0.7 nM ± 0.1 nM and Bmax = 125 ± 42 fmol / mg protein, n = 3), which is consistent with the literature (Neves et al, Bioorg Med Chem 18 (5): 1925-1935, 2010). This Kd value was used to calculate the Ki of the LDT's using the Cheng-Prusoff equation. The Ki obtained values were summarized in the following tables 7 and 8. In Competition assays all tested range was able to inhibit the specific binding of [3H]-8-OH-DPAT. In this receptor, the most prominent derivative was LDT8, which exhibited a very high affinity (Ki = 23.7 pM) compared to it, all other components of the range have significantly lower affinity although all with a high affinity in the nanomolar range.

Compared to LDT62 (Ki = 147 nM), the LTD's 65-68 have 8-29 times higher affinities (Ki (nM) 18, 5, 18 and 9, respectively, P <0.05). On the other hand, LDT63, LDT64, LDT69, LDT70 and LDT39 have similar affinities, with no statistically significant difference (P> 0.05) between values of Ki and that of the LDT62 (Table 8).

Table 7: Pharmacologicalparameters ofLDT's (2-6, 8, 245) for 5-HT1A receptors.

| LDT | n | *log CI50* ± **EPM** *(M)* a | *Ki (M) b* |
|---|---|---|---|
| 2 | 4 | -8,52 ± 0,03 ** | $1,24 \times 10^{-9}$ |
| 3 | 4 | -8,56 ± 0,07 ** | $1,13 \times 10^{-9}$ |
| 4 | 4 | -8,62 ± 0,13 ** | $9,88 \times 10^{-10}$ |
| 5 | 4 | -8,21 ± 0,05** | $2,54 \times 10^{-9}$ |
| 6 | 4 | -9,23 ± 0,24 * | $2,42 \times 10^{-10}$ |
| 8 | 3 | -10,24 ± 0,71 | $2,37 \times 10^{-11}$ |
| 245 | 2 | -8,03 ± 0,05 | $3,80 \times 10^{-9}$ |

a IC50 values were calculated by non-linear regression using GraphPad Prism software (USA) and expressed as mean ± EPM;
b Ki values were calculated using the Cheng-Prusoff equation;
* P <0.01 and ** P <0.001 compared to LDT 8 (single factor ANOVA followed by Newman-Keuls test).

Table 8: Pharmacological Parameters LDT' s (62-70, 39) in the 5-HT1A.

| LDT | 5-HT1A (*n*) | |
|---|---|---|
| | log IC50 (M) | *Ki* (nM) |
| 62 | -6,47 ± 0,10 | 147 (3) |
| 63 | -6,37 ± 0,03 | 191 (3) |
| 64 | -6,72 ± 0,19 | 84 (3) |

(continued)

| LDT | 5-HT1A (*n*) | |
|---|---|---|
| | log IC50 (M) | *Ki* (nM) |
| 65 | -7,42 ± 0,09c | 18 (3) |
| 66 | -7,93 ± 0,20a | 5 (4) |
| 67 | -7,42 ± 0,19b | 18 (3) |
| 68 | -7,70 ± 0,30b | 9 (3) |
| 69 | -6,86 ± 0,30 | 62 (3) |
| 70 | -6,15 ± 0,08 | 316 (4) |
| 39 | -5,93 ± 0,13 | 520 (3) |
| IC50 values were calculated by non-linear regression using GraphPad Prism software (USA) and expressed as mean ± EPM;<br>Ki values were calculated using the Cheng-Prusoff equation;<br>P <0.001, b P <0.01 and c p <0.05 compared to LDT 62 (single-factor ANOVA followed by Newman-Keuls test). | | |

In another competition assay for 5-HT1A receptors, the LDT's 65-68 were capable of specific binding of the antagonist [3H]p-MPPF affinity in the nM range, as well as the agonist [3H]-8-OH-DPAT. The ratios of Ki values for the low and high affinity receptor states, by the agonist and antagonist, respectively, were close to unity (Table 9), suggesting that these four would derivatives act as antagonists of 5-HT1A receptors, which can be useful in inhibiting proliferation of prostate tissue occurring in BPH.

Table 9. intrinsic activity estimative of LDT 65-68 in 5-HT1A receptors.

| LDT | ***Ki* high (nM)** | ***Ki* low (nM)** | ***Ki* low / *Ki* high** |
|---|---|---|---|
| 65 | 18 | 21,2 | 1,18 |
| 66 | 5 | 9,0 | 1,80 |
| 67 | 18 | 6,7 | 0,37 |
| 68 | 9 | 3,1 | 0,34 |

Ki values were calculated using competition experiments with the agonist radioligand [3H] 8-OH-DPAT (high Ki) and the antagonist radioligand [3H] p-MPPF in the presence of GTP (lower Ki) in rat hippocampal membrane preparations. The ratio of Ki for the states of low and high affinity receptor is an estimate of intrinsic activity.

5-HT2A - binding percentage of [3H]-ketanserin

Previously it was performed by our group, saturation assays in rat cortex, enriched tissue in 5-HT2A receptors (Neves et al., Bioorg. Med. Chem. 18 (5): 1925-1935, 2010), in which the determined connection parameters[3H]-ketanserin, (Kd = 1.77 ± 0.07 nM and Bmax = 348 ± 51 fmol / mg protein, n = 2).

This Kd values was used for the calculation of the LDT's Ki using the Cheng-Prusoff equation (Cheng & Prusoff, Biochem. Pharmacol. 22:3099-3108, 1973). The obtained Ki values are summarized in the following Tables 10 and 11. Competition assays in the whole tested range was able to inhibit the specific binding of [3H]-ketanserin.

Table 10: Pharmacological Parameters LDT's (2-6 and 8) with 5-HT2A receptors.

| LDT | n | *log CI50* ± **EPM** *(M)* a | *Ki (M) b* |
|---|---|---|---|
| 2 | 3 | -7,13 ± 0,11 | 4,74 x $10^{-8}$ |
| 3 | 3 | -7,15 ± 0,22 | 4,52 x $10^{-8}$ |
| 4 | 3 | -7,16 ± 0,06 | 4,42 x $10^{-8}$ |

(continued)

| LDT | n | *log CI50* ± **EPM** *(M)* a | *Ki (M) b* |
|---|---|---|---|
| 5 | 3 | -6,41 ± 0,02 | 2,48 x $10^{-7}$ |
| 6 | 3 | -6,23 ± 0,46 | 3,76 x $10^{-7}$ |
| 8 | 3 | -6,41 ± 0,70 | 2,48 x $10^{-7}$ |
| a CI50 values were calculated by non-linear regression using GraphPad Prism software (USA) and expressed as mean ± EPM;<br>b Ki values were calculated using the Cheng-Prusoff equation | | | |

Table 11: pharmacological parameters of the LDT's (62-70, 39) on 5-HT2A receptors.

| | 5-HT2A (*n*) | |
|---|---|---|
| **LDT** | log IC50 (M) | *Ki* (nM) |
| 62 | -5,00 ± 0,16 | 6,41 (3) |
| 63 | -4,86 ± 0,13 | 8,85 (4) |
| 64 | -5,06 ± 0,09 | 5,58 (3) |
| 65 | -5,34 ± 0,09 | 2,93 (4) |
| 66 | -5,57 ± 0,16b | 1,72 (3) |
| 67 | -5,53 ±0,13b | 1,89 (3) |
| 68 | -5,64 ± 0,08b | 1,47 (3) |
| 69 | -5,02 ± 0,06 | 6,12(3) |
| 70 | -4,20 ± 0,03a | 40,4 (3) |
| 39 | -5,16 ± 0,16 | 4,44 (3) |
| CI50 values were calculated by non-linear regression using GraphPad Prism software (USA) and expressed as mean ± EPM;<br>Ki values were calculated using the Cheng-Prusoff equation;<br>P <0.001, b P <0.05 compared to LDT62 (single factor ANOVA followed by Newman-Keuls). | | |

Differently than was observed for the 5-HT1A receptors, there is little difference in affinity between the most LDT's (the difference does not exceed 20 times between the Ki values), except with a much lower affinity LDT70 with lower affinity which has 6 times lower than LDT62 (P <0.05). Generally, compared to affinities of LDT's 2-6 and 8, LTD's 62-70 and LDT39 by all the studied receptors, the observed affinities for 5-HT2A receptors are the smallest, as occurs for the derivative with respect to LDT65 α2A adrenergic receptors.

*α2A receptors - Percentage of binding of [3H]RX821002*

In competition assays the LDT's were able to inhibit the specific binding of [3H]-RX821002 to the α2A adrenergic receptor, but with lower affinities (μM range, Table 12). The fact that these substances present a low affinity for this receptor is important that there is a high homology between α2A adrenoceptor subtypes. Thus, the tested LDT's are likely to present little blocking of this α2A adrenoceptor which is involved with the regulation of central cardiovascular and nervous male genitourinary systems, by inhibiting the release of catecholamines in these systems, reducing the likelihood of adverse effects.

Table 12: Pharmacological parameters of the LDT's on α2A receptors.

| LDT | α 2A | |
|---|---|---|
| | -log IC50 (M) | *Ki* (μM) (n) |
| 3 | 5,96 ± 0,09 | 0,93 |
| 5 | 6,53 ± 0,05 | 0,24 |
| 8 | 6,22 ± 0,12 | 0,55 |
| 245 | 5,7 ± 0,2 | 0,16 |

CI50 values were calculated by non-linear regression using GraphPad Prism software (USA) and expressed as mean ± EPM (n) individual experiments; Ki values were calculated using the Cheng-Prusoff equation.

*5-HT1A receptors - intrinsic activity*

The Ki value ratios for the states of low and high affinity of the receptor, by antagonist and agonist respectively, were close to the unity for the LDT's 3 and 8 (Table 13), suggesting that these derivatives are 5-HT1A antagonist receptor. Therefore they can be useful in the proliferation inhibition of the prostate tissue that occurs in BPH.

Table 13. Intrinsic activity estimate of LDT's on 5-HT1A receptors.

| LDT | *Ki* high (nM) | *Ki* low (nM) | *Ki* low / *Ki* high | Pharmacological classification |
|---|---|---|---|---|
| **3** | **1,79** | **1,99** | **1,1 [0,6-2,2]** | **antagonist** |
| **5** | **3,89** | **8,12** | **2,09 [1,3-3,5]** | **antagonist** |
| **8** | **0,018** | **0,62** | **34,4 [3,7-186]*** | **partial agonist** |
| **5-HT** | **2,46** | **189** | **76,8 [45-146]*** | **full agonist** |
| Ki values were calculated by competition experiments with agonist radioligand [3H] 8-OH-DPAT (high Ki) and the antagonist radioligand [3 H] p-MPPF in the presence of GTP (lower Ki) in membrane preparations from rat hippocampus. CI = confidence interval. 5-HT used as a positive control.<br>* Different from 1.0 (P <0.05, Student's t test), | | | | |

*Other non-receptor targets for the treatment of BPH*

G-protein coupled receptors have structural similarities that difficult the obtaining of selective ligands (reviewed in Oldham & Hamm, Nature 9:60-71, 2008). Additionally, blocking non-target receptors for the disease in question is related to an increased likelihood of adverse effects. Thus, in addition to α2 adrenoceptor (already presented in this text, Table 12), we also conducted the investigation of the action of LDT's on muscarinic and dopaminergic D2 receptors. All LDT's showed a lower affinity for these receptors, and particularly LDT3 and LDT5 with Ki in micromolar range (Tables 14 and 15) in relation to the target receptors, i.e. α1A, α1D adrenoceptor and 5-HTIA receptors. Therefore these LDT's should have little propensity for adverse effects.

Table 14: Pharmacological Parameters LDT's on muscarinic receptors.

| LDT | muscarinic | |
|---|---|---|
| | -log IC50 (M) | *Ki* (μM) (n) |
| 3 | 4,26 ± 0,07 | 56,7 (3) |
| 5 | 3,97 ±0,06 | 108 (3) |
| 8 | 4,48 ± 0,07 | 33,9 (3) |
| 65 | 3,3 ± 0,08 | 172 (3) |
| 66 | 3,8 ± 0,08 | 54,2 (3) |
| 67 | 4,2 ±0,02 | 21 (3) |

(continued)

| LDT | muscarinic | |
|---|---|---|
| | -log IC50 (M) | *Ki* (μM) (n) |
| 68 | 4,5 ±0,05 | 10,8 (3) |

IC50 values were calculated by non-linear regression using GraphPad Prism software (USA) and expressed as the mean ± EPM of (n) individual experiments; Ki values were calculated using the Cheng-Prusoff equation.

Table 15: pharmacological parameters of LDT's on D2 dopaminergic receptors.

| LDT | D2 | |
|---|---|---|
| | -log IC50 (M) | *Ki* (μM) (n) |
| 3 | 6,73 ± 0,13 | 0,186 (3) |
| 5 | 7,05 ± 0,04 | 0,1 (3) |
| 8 | 7,92 ± 0,25 | 0,012 (3) |
| 245 | 7,91 ± 0,01 | 0,004 (3) |
| 65 | 6,88 ± 0,05 | 0,04 (4) |
| 66 | 6,92 ± 0,11 | 0,04 (4) |
| 67 | 7,47 ± 0,07 | 0,009 (4) |
| 68 | 7,49 ± 0,09 | 0,009 (4) |

IC50 values were calculated by non-linear regression using GraphPad Prism software (USA) and expressed as the mean ± EPM of (n) individual experiments; Ki values were calculated using the Cheng-Prusoff equation.

*Proliferation assays using human prostate cells in vitro*

Literature data show that prostate cells expressing the α1A adrenoceptor, α1D adrenoceptor and 5HT1A receptor, wherein α1D adrenoceptor and 5HT1A receptor are related to cell proliferation (DIZEYI et al Prostate 59 (3):328-336, 2004; KOJIMA and cols., Prostate 66:761-767, 2006). In these models, tamsulosin does not inhibit cell proliferation (DIZEYI et al. Prostate 59 (3) :328-336, 2004).

The analysis of our data showed that the LDT's used in a nanomolar range of concentration in vitro inhibits the stimulation of tumor-origin human prostatic cell. In particular, LDT3 (50 nM) and LDT66 (20 nM) inhibited the effect in face of the α1D receptors and 5-HT1A receptors stimulus (Table 16).

Table 16. LDT's effect on the in vitro proliferation of human prostate cells

| LDT | | | | |
|---|---|---|---|---|
| | Proliferation percentage in relation to basal (100%) | | Proliferation percentage in relation to basal (100%) in the presence of LDT's | |
| | α 1D | 5-HT1A | α 1D | 5-HT1A |
| 3 (50[b] ou 500[a] nM) | - | | 99,2 ± 5,8[a] | 96,9 ± 5,8[b] |
| 5 (50 nM) | - | | 117,3 ± 8,9 | 118,8 ± 9,9 |
| 8 (5 nM) | - | | 124,05 ± 9,6 | 138,4 ± 42,9 |
| 66 (50 nM) | -<br>-<br>- | | 76 ± 7,7[a] | 110,8 ± 7,7[b] |

(continued)

| LDT | | | | |
|---|---|---|---|---|
| | Proliferation percentage in relation to basal (100%) | | Proliferation percentage in relation to basal (100%) in the presence of LDT's | |
| | α 1D | 5-HT1A | α 1D | 5-HT1A |
| 5-HT (1 μM) | ND | 137,1 ± 5,2 | | |
| Phenylephrine (3μM) | 138,8 ± 2,6 | ND | | |
| BMY7378 (50 nM) | 90,98 ± 11,7[a] | ND | | |
| p-MPPF (50 nM) | ND | 88,9 ± 11,5[b] | | |

5-HT (1 μM) and phenylephrine (3 μM) were used as stimulating the α1D receptors and 5-HT1A receptors. BMY7378 (50 nM) and p-MMPF (50 nM) are selective antagonists of the α1D receptors and 5-HT1A receptors and were used as positive controls.
Experiments performed in five times. N = 3.
a condition in face for phenylephrine, b condition in face of 5-HT.
a,b P <0.05 versus phenylephrine and 5-HT, respectively. ND = not determined.

*Tests for acute toxicity study*

In the used dose (100 mg / kg ip), ten times higher than the usual dose in this type of study, there was no death of animals or temperature change (n = 6 animals / condition) (Table 17).

Table 17. Evaluation of the effect of LDT's on body temperature.

| | Control | | | LDT 3 | | | LDT 5 | | | LDT 8 | | |
|---|---|---|---|---|---|---|---|---|---|---|---|---|
| Basal Measure | T (°C) | EPM | n | T (°C) | EPM | n | T (°C) | EPM | n | T (°C) | EPM | n |
| | 35,17 | 0,17 | 6 | 35,33 | 0,33 | 6 | 35,00 | 0,26 | 6 | 35,67 | 0,21 | 6 |
| 30' after LDT | 35,50 | 0,22 | 6 | 35,50 | 0,22 | 6 | 35,50 | 0,22 | 6 | 35,67 | 0,21 | 6 |
| 60' after LDT | 35,50 | 0,23 | 6 | 35,50 | 0,22 | 6 | 35,17 | 0,31 | 6 | 35,50 | 0,22 | 6 |
| | **Control** | | | **LDT 65** | | | **LDT 66** | | | | | |
| Basal Measure | T (°C) | EPM | n | T (°C) | EPM | n | T (°C) | EPM | n | | | |
| | 36,33 | 0,56 | 6 | 37,17 | 0,31 | 6 | 37,00 | 0,26 | 6 | | | |
| 30' after LDT | 36,83 | 0,31 | 6 | 37,17 | 0,17 | 6 | 37,50 | 0,22 | 6 | | | |
| 60' after LDT | 37,17 | 0,48 | 6 | 37,17 | 0,17 | 6 | 37,33 | 0,42 | 6 | | | |

The blocking of the receptors at central level could cause symptoms as described in Table 8. Considering the parameters described in Table 8, none of LDT's (100 mg / kg ip) caused no effect in 14 days, thus ruling out acute toxicity. Importantly, the reduced affinity of LDT's by non target receptors (Tables 2, 4 and 5) contributes to the non-observation of adverse effects (Table 9).

Table 18. Adverse effects resulting from central blocking of G-protein coupled receptors

| Receptor | Antagonist adverse effect |
|---|---|
| α1 adrenoceptor | Hypotension, sedation, dizziness |
| α2 adrenoceptor | Anxiety |
| 5-HT1A | temperature changes |

(continued)

| Receptor | Antagonist adverse effect |
|---|---|
| D2/D3 | catalepsy |
| D1-like, D4 | control of urination chances |
| muscarinic | sedation |

Table 19. Behavioral parameters evaluated for 14 days after treatment with LDT's.

| Parameters | Evaluation forms |
|---|---|
| care state and welfare | Appearance, irritability |
| coordination | activity, reply to the touch, reply to constriction of the tail, abdominal contraction, hiking, reflex stiffness |
| Muscle tone | - |
| Activity of the central nervous system | tremors, convulsion, hyperactivity, sedation, hypnosis and anesthesia |
| Activity of the autonomous nervous system | tears, urination, defecation, piloerection, hypothermia and breathing |
| Consumption of water and food | - |
| Reference: Lorke. Arch Toxicol. 54:275-287. 1983; SOUZA and Deal. J Ethnopharmacol. 135: 135-146, 201 1. | |

**Claims**

**1.** N-phenylpiperazine derivatives with high affinity for α1A /D adrenoceptors and 5-HT1A receptors comprising:

- a basic nitrogen atom for ionic interaction with the carboxylate group of the aspartate residue (Asp) in TM3 of these receptors; and
- at least one aromatic ring for hydrophobic and electrostatic interactions with complementary residues in TM2 4, 6 and 7; and
- a group with a negative charge density at position 2 (*ortho*) to the nitrogen or the N-phenylpiperazine subunit, hydrogen bond acceptor e.g. alkoxides and isosteres.

**2.** N-phenylpiperazine derivatives according to claim 1 selected from the group comprising:

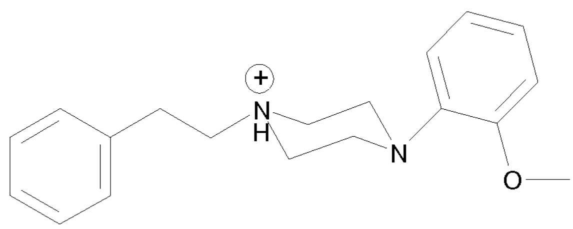

LDT 2
1-(2-phenylethyl)-4-(2-methoxyphenyl)piperazine

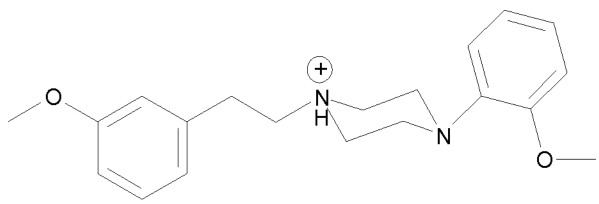

LDT 3
1-(2-methoxyphenyl)-4-[2-(3-methoxyphenyl)ethyl] piperazine

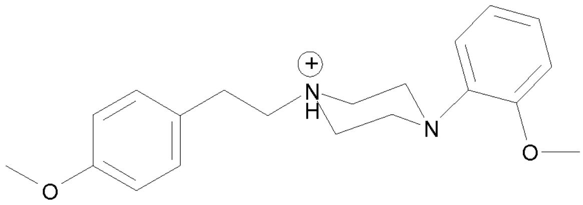

LDT 4

1-(2-methoxyphenyl)-4-[2-(4-methoxyphenyl)ethyl] piperazine

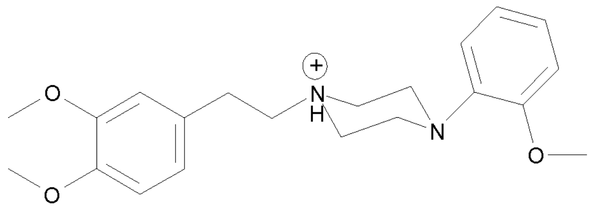

LDT 5

1-(2-methoxyphenyl)-4-[2-(3,4-dimethoxyphenyl)ethyl] piperazine

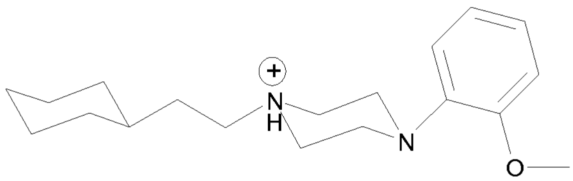

LDT 6

1-(2-cyclohexylethyl)-4-(2-methoxyphenyl) piperazine

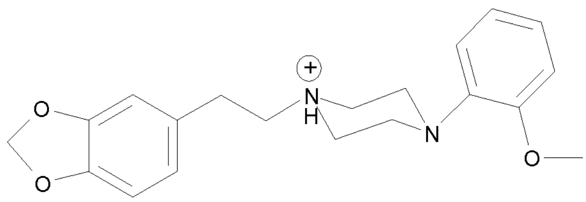

LDT 8

1-(1,3-benzodioxol-5-ilethyl) -4-(2-ethoxyphenyl)piperazine

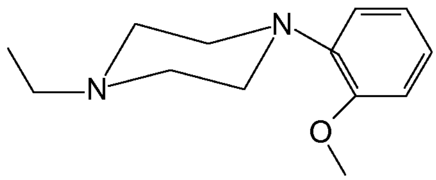

LDT 62

1-ethyl-4-(2-methoxyphenyl) piperazine

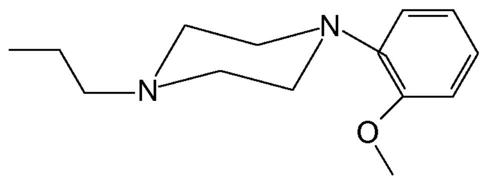

LDT 63

1-(2-methoxyphenyl)-4-propylpiperazine

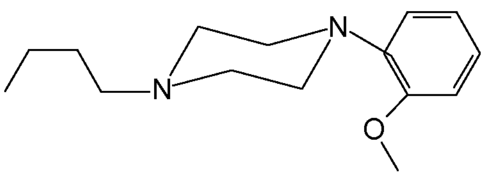

LDT 64

1-butyl-4-(2-methoxyphenyl) piperazine

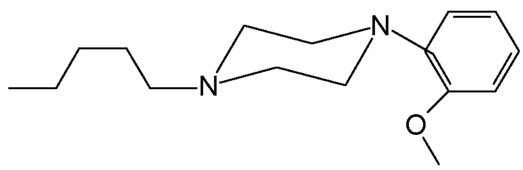

LDT 65

1-(2-methoxyphenyl)-4- pentyl piperazine

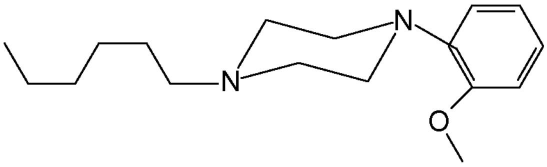

LDT 66

1-hexyl-4-(2-methoxyphenyl) piperazine

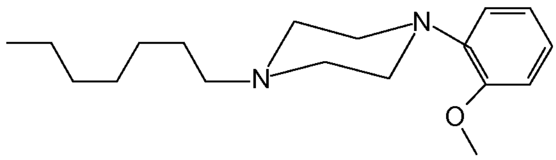

LDT 67

1-heptyl-4-(2-methoxyphenyl) piperazine

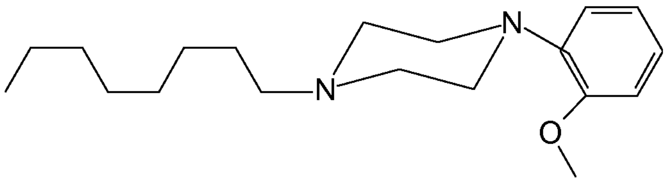

LDT 68

1- (2-methoxyphenyl)-4-octylpiperazine

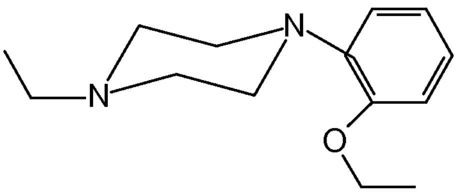

LDT 69

1-ethyl-4-(2-ethoxyphenyl) piperazine

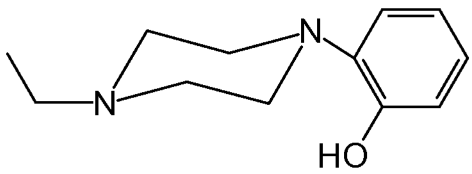

LDT 70

1-ethyl-4-(2-hydroxyphenyl) piperazine

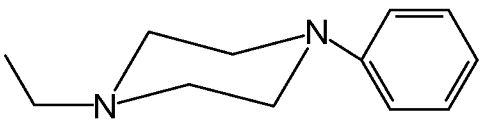

LDT 39

1- ethyl-4-(2-hydroxyphenyl)piperazine

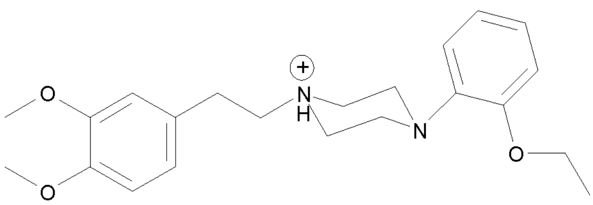

LDT 245

1 (-2-ethoxyphenyl)-4-[2-(3,4-dimethoxy)ethyl]piperazine

**3.** α1A /D adrenoceptors and 5-HT1A receptors ligands comprising:

- a basic nitrogen atom for ionic interaction with the carboxylate group of the aspartate residue (Asp) in TM3 of these receptors; and
- at least one aromatic ring for hydrophobic and electrostatic interactions with complementary residues in TM2 4, 6 and 7.

4. Ligands according to claim 3 selected from the group comprising:

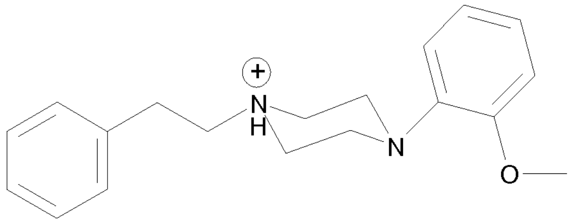

LDT 2

1-(2-phenylethyl)-4-(2-methoxyphenyl)piperazine

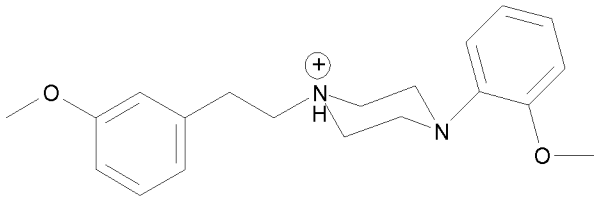

LDT 3

1-(2-methoxyphenyl)-4-[2-(3-methoxyphenyl)ethyl] piperazine

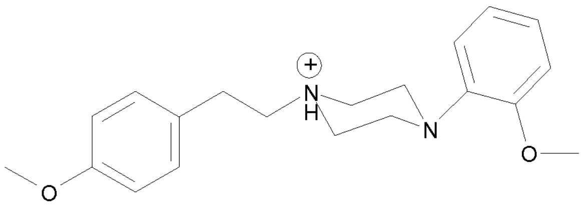

LDT 4

1-(2-methoxyphenyl)-4-[2-(4-methoxyphenyl)ethyl] piperazine

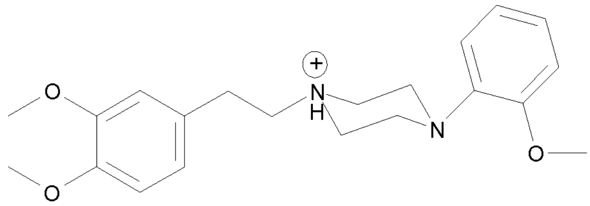

LDT 5

1-(2-methoxyphenyl)-4-[2-(3,4-dimethoxyphenyl)ethyl] piperazine

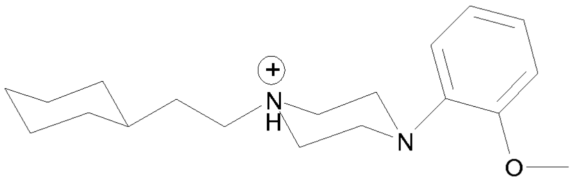

LDT 6

1-(2-cyclohexylethyl)-4-(2-methoxyphenyl) piperazine

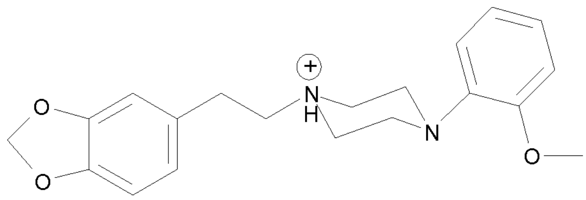

LDT 8

1-(1,3-benzodioxol-5-ilethyl) -4-(2-ethoxyphenyl)piperazine

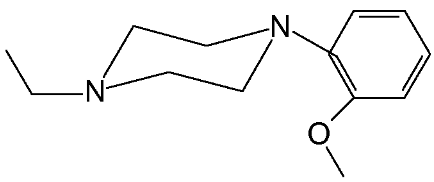

LDT 62

1-ethyl-4-(2-methoxyphenyl) piperazine

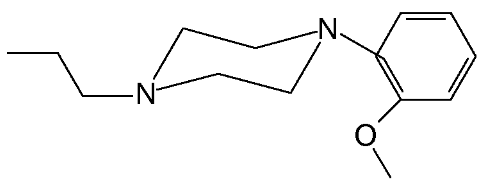

LDT 63

1-(2-methoxyphenyl)-4-propylpiperazine

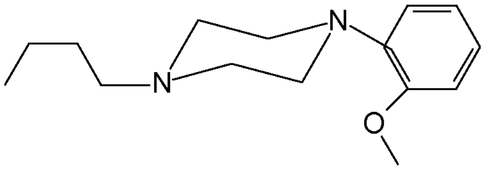

LDT 64

1-butyl-4-(2-methoxyphenyl) piperazine

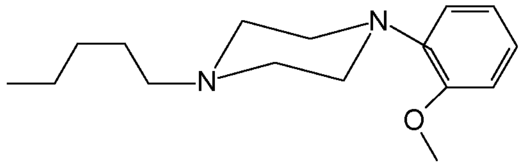

LDT 65

1-(2-methoxyphenyl)-4- pentyl piperazine

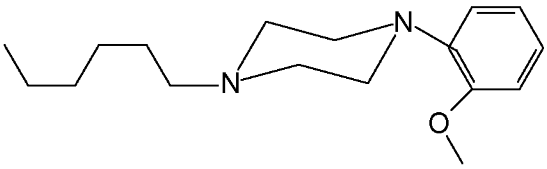

LDT 66

1-hexyl-4-(2-methoxyphenyl) piperazine

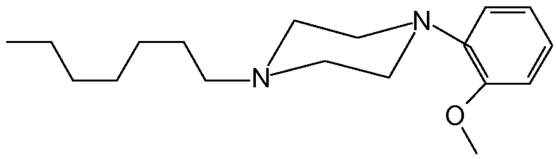

LDT 67

1-heptyl-4-(2-methoxyphenyl) piperazine

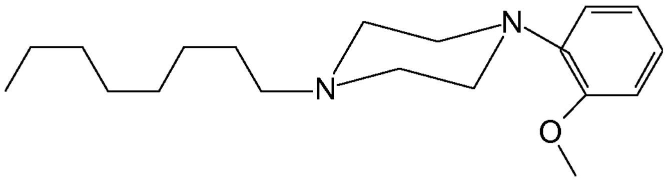

LDT 68

1- (2-methoxyphenyl)-4-octylpiperazine

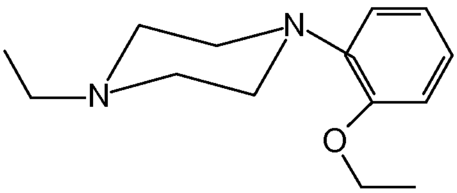

LDT 69

1-ethyl-4-(2-ethoxyphenyl) piperazine

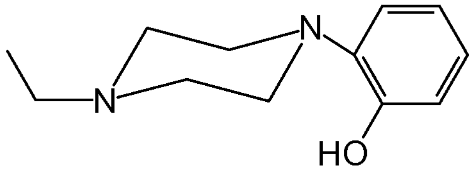

LDT 70

1-ethyl-4-(2-hydroxyphenyl) piperazine

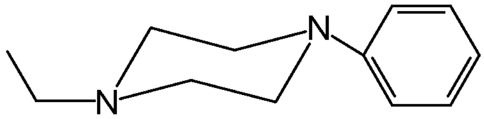

LDT 39

1- ethyl-4-(2-hydroxyphenyl)piperazine

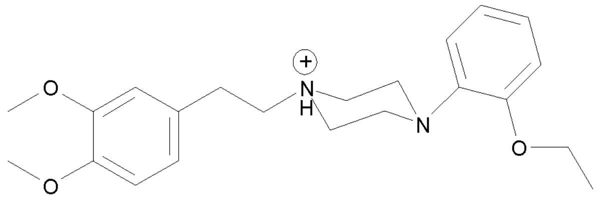

LDT 245

1 (-2-ethoxyphenyl)-4-[2-(3,4-dimethoxy)ethyl]piperazine

**5.** Pharmaceutical composition for the treatment of benign prostatic hyperplasia and lower urinary tract symptoms and prostatic anti-proliferative effect comprising a pharmaceutically acceptable carrier and at least one N-phenylpiperazine derivatives with high affinity for $\alpha$1A /D adrenoceptors and 5-HT1A receptors comprising:

- a basic nitrogen atom for ionic interaction with the carboxylate group of the aspartate residue (Asp) in TM3 of these receptors; and
- at least one aromatic ring for hydrophobic and electrostatic interactions with complementary residues in TM2 4, 6 and 7.

**6.** Composition according to claim 5 wherein the said derivative is selected from the group comprising:

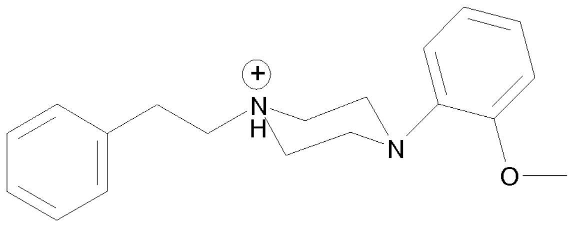

LDT 2

1-(2-phenylethyl)-4-(2-methoxyphenyl)piperazine

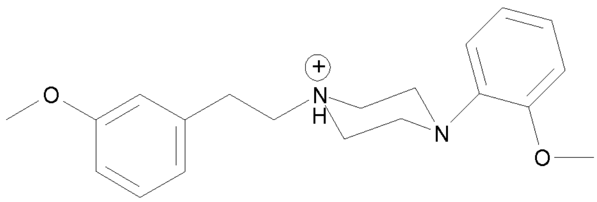

LDT 3

1-(2-methoxyphenyl)-4-[2-(3-methoxyphenyl)ethyl] piperazine

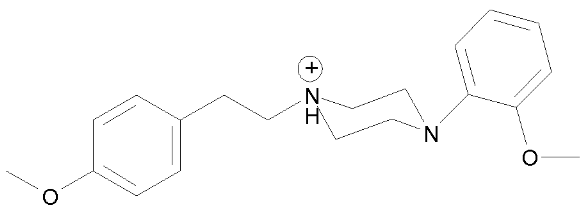

LDT 4

1-(2-methoxyphenyl)-4-[2-(4-methoxyphenyl)ethyl] piperazine

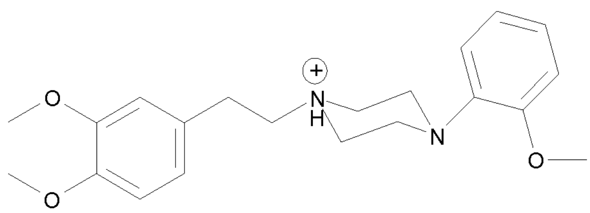

LDT 5

1-(2-methoxyphenyl)-4-[2-(3,4-dimethoxyphenyl)ethyl] piperazine

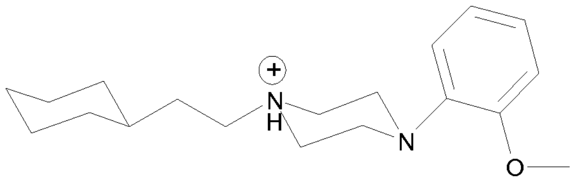

LDT 6

1-(2-cyclohexylethyl)-4-(2-methoxyphenyl) piperazine

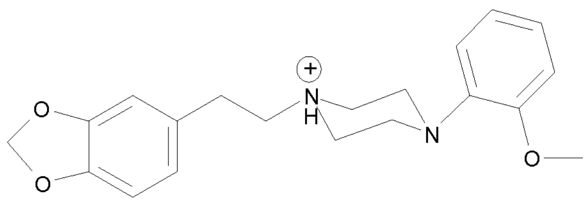

LDT 8

1-(1,3-benzodioxol-5-ilethyl) -4-(2-ethoxyphenyl)piperazine

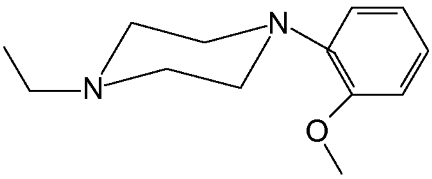

LDT 62

1-ethyl-4-(2-methoxyphenyl) piperazine

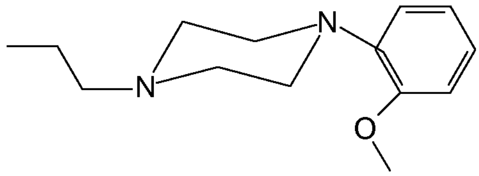

LDT 63

1-(2-methoxyphenyl)-4-propylpiperazine

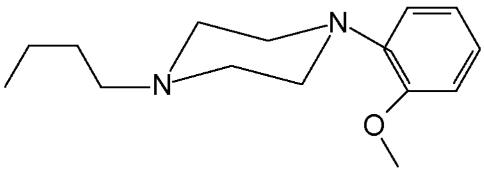

LDT 64

1-butyl-4-(2-methoxyphenyl) piperazine

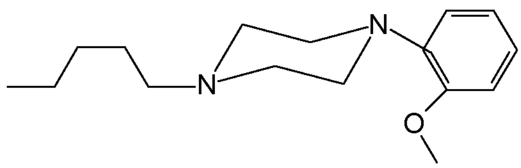

LDT 65

1-(2-methoxyphenyl)-4- pentyl piperazine

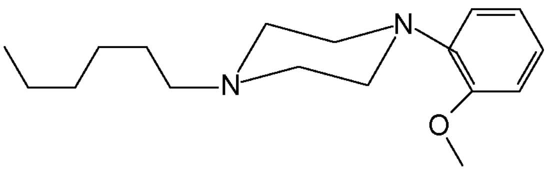

LDT 66

1-hexyl-4-(2-methoxyphenyl) piperazine

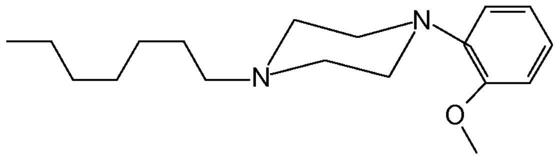

LDT 67

1-heptyl-4-(2-methoxyphenyl) piperazine

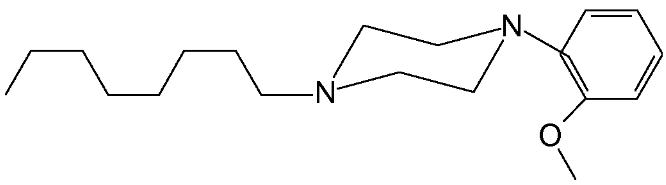

LDT 68

1- (2-methoxyphenyl)-4-octylpiperazine

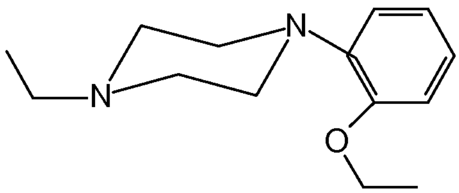

LDT 69

1-ethyl-4-(2-ethoxyphenyl) piperazine

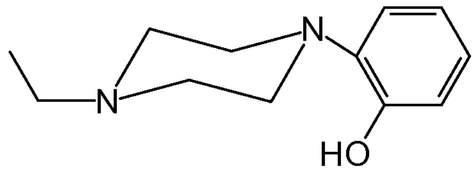

LDT 70

1-ethyl-4-(2-hydroxyphenyl) piperazine

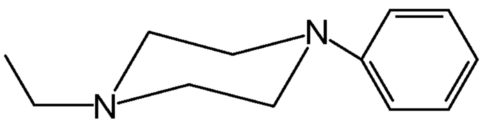

LDT 39

1- ethyl-4-(2-hydroxyphenyl)piperazine

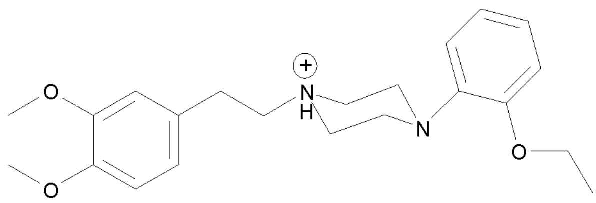

LDT 245

1 (-2-ethoxyphenyl)-4-[2-(3,4-dimethoxy)ethyl]piperazine

Figure 1

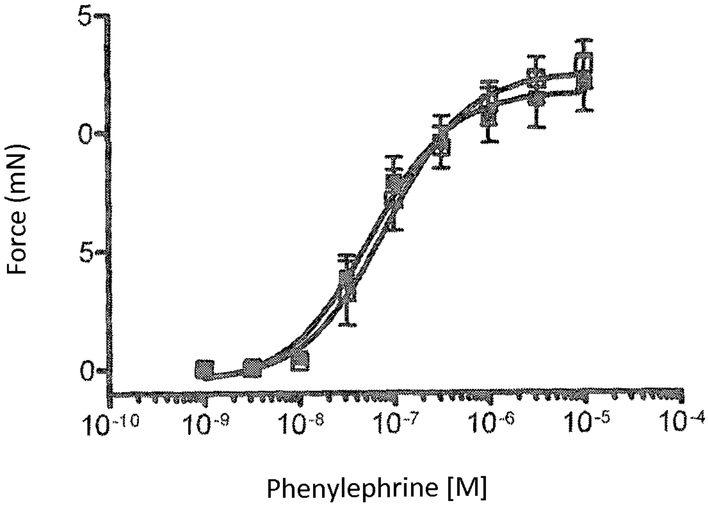

**INTERNATIONAL SEARCH REPORT**

International application No. PCT/BR2013/000003

A. CLASSIFICATION OF SUBJECT MATTER

**C07D219/00 (2006.01), A61K31/473 (2006.01), A61P35/00 (2006.01).**

According to International Patent Classification (IPC) or to both national classification and IPC

B. FIELDS SEARCHED

Minimum documentation searched (classification system followed by classification symbols)

**C07D, A61K, A61P**

Documentation searched other than minimum documentation to the extent that such documents are included in the fields searched

**Banco de Patentes Brasileiro (INPI-BR)**

Electronic data base consulted during the international search (name of data base and, where practicable, search terms used)

**Epodoc, WPI, Medline, Espacenet**

C. DOCUMENTS CONSIDERED TO BE RELEVANT

| Category* | Citation of document, with indication, where appropriate, of the relevant passages | Relevant to claim No. |
|---|---|---|
| X<br>Y | WO 9504049 A1 ( TESTA RODOLFO [IT]) (todo documento)<br>09 **Feb.** 1995 (1995-02-09) | 1, 3, 5<br>2,4,6 |
| Y | JP H0324014 A (BOEHRINGER MANNHEIM GMBH)<br>01 **Feb.** 1991 (1991-02-01) **(abstract)** | 2,4,6 |
| Y | JP H09227380 A (MITSUBISHI CHEM CORP)<br>02 **Sept.** 1997 (1997-09-02) **(abstract)** | 2,4,6 |
| Y | Pulito VL, Li X, Varga SS, et al. An Investigation of the Uroselective Properties of Four Novel alfa1a-Adrenergic Receptor Subtype-Selective Antagonists. J Pharm Exp Ther. 2000,292(1):224-229. **(abstract)** | 2,4,6 |

[X] Further documents are listed in the continuation of Box C. [X] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search: **24 April 2013**

Date of mailing of the international search report: **20 May 2013**

Name and mailing address of the ISA/ BR
INPI
INSTITUTO NACIONAL DA PROPRIEDADE INDUSTRIAL
Rua Sao Bento nº 1, 17º andar
cep: 20090-010, Centro - Rio de Janeiro/RJ

Authorized officer: **Thiago Silva Torres**

Telephone No. +55 21 3037-3984/3742

Form PCT/ISA/210 (second sheet) (July 2009)

**INTERNATIONAL SEARCH REPORT**

International application No.
PCT/BR2013/000003

C (Continuation). DOCUMENTS CONSIDERED TO BE RELEVANT

| Category* | Citation of document, with indication, where appropriate of the relevant passages | Relevant to claim No. |
|---|---|---|
| A | WO 03043983 A1 (SCHERING AG [DE]) 30 May 2003 (2003-05-30) | - |
| A | Bradbury RH, Hales NJ, Rabow AA, et al. Small-molecule androgen receptor downregulators as an approach to treatment of advanced prostate cancer. Bioorg Med Chem Letters 2011,21:5442-5445. | - |
| A | Kumar CSA, Prasad SBB, Vinaya K, et al. Synthesis and in vitro antiproliferative activity of novel 1-benzhydrylpiperazine derivatives against human cancer cell lines. Eur J Med Chem 2009, 44:1223-1229. | - |

Form PCT/ISA/210 (continuation of second sheet) (July 2009)

**INTERNATIONAL SEARCH REPORT**
Information on patent family members

International application No.
PCT/BR2013/000003

| | | | |
|---|---|---|---|
| WO 9504049 A1 | 1995-02-09 | AU 680037 B2 | 1997-07-17 |
| | | AU 7532394 A | 1995-02-28 |
| | | CA 2168443 A1 | 1995-02-09 |
| | | CN 1132508 A | 1996-10-02 |
| | | EP 0711288 A1 | 1996-05-15 |
| | | IL 110348 D0 | 1994-10-21 |
| | | IT MI931717 D0 | 1993-07-30 |
| | | IT 1266582 B1 | 1997-01-09 |
| | | JP H09500883 A | 1997-01-28 |
| | | MX PA94005805 A | 2004-08-20 |
| | | NO 960371 D0 | 1996-01-29 |
| | | NZ 271634 A | 1996-09-25 |
| | | SG 46281 A1 | 1998-02-20 |
| | | ZA 9405625 A | 1995-03-07 |
| JP H0324014 A | 1991-02-01 | AT 84416 T | 1993-01-15 |
| | | AU 625766 B2 | 1992-07-16 |
| | | AU 5628290 A | 1990-12-13 |
| | | CA 2017619 A1 | 1990-12-07 |
| | | DD 294864 A5 | 1991-10-17 |
| | | DE 3918543 A1 | 1990-12-13 |
| | | DE 59000748 D1 | 1993-02-25 |
| | | DK 401653 T3 | 1993-03-29 |
| | | ES 2054147 T3 | 1994-08-01 |
| | | GR 3007328 T3 | 1993-07-30 |
| | | HK 46996 A | 1996-03-22 |
| | | HU 903515 D0 | 1990-10-28 |
| | | HU T54494 A | 1991-03-28 |
| | | HU 206976 B | 1993-03-01 |
| | | IE 902014 L | 1990-12-07 |
| | | IE 62937 B1 | 1995-03-08 |
| | | IL 94601 D0 | 1991-04-15 |
| | | JP H062673 B2 | 1994-01-12 |
| | | KR 0131452 B1 | 1998-04-17 |
| | | NZ 245673 A | 1997-05-26 |
| | | PH 26690 A | 1992-09-15 |
| | | PT 94268 A | 1991-02-08 |
| | | US 5026706 A | 1991-06-25 |
| | | ZA 9004338 A | 1991-03-27 |
| JP H09227380 A | 1997-09-02 | **None** | |
| WO 03043983 A1 | 2003-05-30 | AU 2002360932 A1 | 2003-06-10 |
| | | DE 10159035 A1 | 2003-06-12 |
| | | DE 10238742 A1 | 2004-03-04 |
| | | PE 07082003 A1 | 2003-08-28 |
| | | US 2004009969 A1 | 2004-01-15 |
| | | US 6861432 B2 | 2005-03-01 |
| | | UY 27544 A1 | 2003-06-30 |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **OLDHAM ; HA MM.** G protein-coupled receptors. *Nature,* 2008, vol. 9, 60-71
- **FREDRIKSSON et al.** *Mol. Pharmacol.,* 2003, vol. 63, 1256-1272
- **OVERINGTON.** *Nat. Rev. Drug Discov.,* 2006, vol. 5 (12), 993-996
- **BJARNADOTTIR RN et al.** *Genomics,* 2006, vol. 88, 263-273
- **OLDHAM ; HAMM.** *Nature,* 2008, vol. 9, 60-71
- **AVELLAR et al.** *An. Acad. Bras. Cienc.,* 2009, vol. 81 (3), 321-344
- **WESS.** *Pharmacol. Ther.,* 1998, vol. 80 (3), 231-264
- **FREDRIKSSON et al.** *Mol Pharmacol,* 2003, vol. 63, 1256-1272
- **HIEBLE et al.** *Pharmacol Rev,* 1995, vol. 45, 267-270
- **MICHEL et al.** *Naunyn Schmiedebergs Arch Pharmacol,* vol. 352 (1), 1-10
- **LANGER.** *Eur. J. Urol,* 1999, vol. 36, 2-6
- **VARMA ; DENG.** *Can. J. Physiol. Pharmacol.,* 2000, vol. 78, 267-292
- **ZHONG ; MINNEMAN.** *Eur. J. Pharmacol.,* 1999, vol. 375, 261-276
- **HUH et al.** *Genes Genet. Syst.,* 2010, vol. 85 (l), 65-73
- **SCOFIELD et al.** *J. Pharmacol. Exp. Ther.,* 1995, vol. 275, 1035-1042
- **HIEBLE.** *Pharm. Acta. Helv.,* 2000, vol. 74 (2-3), 163-171
- **MICHELOTTI et al.** *Pharmacol. Ther.,* 2000, vol. 88, 281-309
- **FORRAY et al.** *Mol. Pharmacol.,* 1994, vol. 45 (4), 703-708
- **HATANO et al.** *Br. J. Pharmacol.,* 1994, vol. 113 (3), 723-728
- **MARSHALL et al.** *J. Pharmacol.,* 1995, vol. 115 (5), 781-786
- **HIEBLE ; RUFFOLO.** *Expert Opin Investig Drugs,* 1997, vol. 6 (4), 367-387
- **MALLOY et al.** *J. Urol.,* September 1998, vol. 160 (3), 937-943
- **MURAMATSU et al.** *Br J. Urol.,* 1994, vol. 74 (5), 572-578
- **HIEBLE et al.** *Pharmacol. Rev.,* 1995, vol. 45, 267-270
- **CHEN et al.** *J. Urol.,* 2005, vol. 174, 370-374
- **MCNEAL.** *Urol. Clin. North Am.,* 1990, vol. 17, 477-486
- **COCKETT et al.** *Prog. Urol.,* 1991, vol. 1 (6), 957-72
- **PRICE et al.** *Mol Pharmacol,* 1994, vol. 46, 221-226
- **TSENG-CRANK et al.** *J. Pharmacol.,* 1995, vol. 115, 1475-1485
- **LEPOR et al.** *J. Urol,* 1993, vol. 149, 640-642
- **MICHEL et al.** *J. Auton Pharmacol,* 1996, vol. 16, 21-28
- **KOJIMA et al.** *Prostate,* 2006, vol. 66, 761-767
- **CHAPPLE.** *Br J Urol,* 1995, vol. 76 (1), 47-56
- **SHIBATA et al.** *Mol. Pharmacol.,* 1995, vol. 48, 250-258
- **GOETZ et al.** *Eur. J. Pharmacol.,* 1995, vol. 272 (2-3), R5-6
- **CHAPUT ; MONTIGNI.** *J. Pharmacol. Ther.,* 1988, vol. 246 (1), 359-370
- **HOYER et al.** *Pharmacol Rev,* 1994, vol. 46, 157-203
- **HOYER ; MARTIN.** *Neuropharmacology,* 1997, vol. 36, 419-428
- **SAXENA et al.** *Trends Pharmacol Sci,* 1998, vol. 19, 311-316
- **VILLALON et al.** *Drug Discov Today,* 1997, vol. 2, 294-300
- **VILLALON ; CENTURION.** *Naunyn. Schmiedebergs Arch Pharmacol,* 2007, vol. 376 (1-2), 45-63
- **FREDRIKSSON et al.** *Mol Pharmacol.,* 2003, vol. 63, 1256-1272
- **TRUMPP-KALLMEYER et al.** *J. Med.,* 1992, vol. 35 (19), 3448-3462
- **BARNES ; SHARP.** *Neuropharmacology,* 1999, vol. 38, 1083-1152
- **KOBILKA et al.** *Nature,* 1987, vol. 329, 75-79
- **FARGIN et al.** *Nature,* 1988, vol. 335 (6188), 358-360
- **PUCADYIL ; CHATTOPADHYAY.** *Progr. Lipid Res.,* 2006, vol. 45, 295-333
- **ABDUL et al.** *Anticancer Res,* 1994, vol. 14 (3A), 1215-1220
- **DIZEYI et al.** *Prostate,* 2004, vol. 59 (3), 328-336
- **ABDUL.** *Anticancer Res,* 1994, vol. 14 (3A), 1215-1220

- **TATE ; SCHERTLER.** *Curr Opin Struct Biol,* 2009, vol. 19 (4), 386-395
- **KOBILKA ; SCHERTLER.** *Trends Pharmacol Sci,* 2008, vol. 29 (2), 79-83
- **THIEL.** *Nat Biotechnol,* 2004, vol. 22, 513-519
- **PEREZ.** *Biochem Pharmacol,* 2007, vol. 73 (8), 1051-1062
- **WAUGH et al.** *J. Biol Chem,* 2001, vol. 276 (27), 25366-2537
- **LOPEZ-RODRIGUEZ et al.** *J. Med. Chem,* 1997, vol. 40, 2653-2656
- **PEREZ.** *Biochem Pharmacol,* 2007, vol. 73 (8), 1051-1062
- **LI et al.** *J. Mol. Model,* 2008, vol. 14 (10), 957-966
- **LOPEZ-RODRIGUEZ et al.** *Mol Pharmacol,* 2002, vol. 62 (1), 15-21
- **KUBINYI.** *Pharmazie,* 1995, vol. 50, 647-662
- **HUBER et al.** *Biochemistry,* 2008, vol. 47 (42), 11013-1 1023
- **FORRAY et al.** *Pharmacol.,* 1994, vol. 45 (4), 703-708
- **HATANO et al.** *J. Pharmacol,* 1994, vol. 113 (3), 723-728
- **MARSHALL et al.** *Br J Pharmacol,* 1995, vol. 115 (5), 1-786 78
- **MALLOY et al.** *Urol, J.,* September 1998, vol. 160 (3), 937-943
- **MURAMATSU et al.** *Br J Urol.,* 1994, vol. 74 (5), 572-578
- **HIEBLE et al.** *Pharmacol Rev.,* 1995, vol. 45, 267-270
- **HIEBLE.** *Pharma Helv Acta,* 2000, vol. 74 (2-3), 163-171
- **MTCHELOTTI et al.** *Pharmacol. Ther.,* 2000, vol. 88, 281-309
- **DIZEYL et al.** *Prostate,* 2004, vol. 59 (3), 328-336
- **RATOUIS et al.** *J. Med.,* 1965, vol. 8, 271-273
- **LEOPOLD et al.** *J Pharm. Pharmacol.,* 2000, vol. 56 (2), 247-255
- **MOKROSZ et al.** *Arch Pharm,* 1995, vol. 328, 143-148
- **MOKROSZ et al.** *J Med Chem,* 1996, vol. 39, 1125-1129
- **KENAKIN.** Schild equation. Raven Press, 1993, 278-322
- **LOWRY et al.** *J. Biol. Chem.,* 1951, vol. 193 (1), 265-275
- **RICHARDS.** *J Pharmacol,* 1990, vol. 99, 753-761
- **CASTOLDI.** *Life Sci.,* 2006, vol. 78, 1915-1924
- **NEVES et al.** *Bioorg Med. Chem.,* 2010, vol. 18 (5), 1925-1935
- **NIZNIK et al.** *Naunyn-Schmiedebergs Arch.. Pharmacol,* 1985, vol. 329, 333-343
- **TERAI et al.** *Eur J Pharmacol,* 1989, vol. 173, 177
- **HAMDI et al.** *Life Sci,* 1992, vol. 50, 1529-1534
- **NEVES et al.** *Bioorg. Med.,* 2010, vol. 18 (5), 1925-1935
- **DE LEAN et al.** *Mol Pharmacol.,* 1982, vol. 21 (1), 5 it
- **MOSMANN, T.** *Immunol Methods.,* 1983, vol. 65 (1 -2), 55-63
- **LORKE, D.** *Arch Toxicol,* 1983, vol. 54 (4), 275-87
- **SCOFIELD et al.** *J. Pharmacol Exp Ther,* 1995, vol. 275, 1035-1042
- **TESTA et al.** *Life Sci,* 1995, vol. 57 (13), 1159-63
- **BANES et al.** *J. Pharmacol Exp Ther,* 1999, vol. 291, 1179-1187
- **CARROLL et al.** *Bioorg Med Chem Lett,* 2001, vol. 11, 1119-1121
- **SHIBATA.** *Mol. Pharmacol.,* 1995, vol. 48, 250-258
- **NEVES et al.** *Bioorg Med Chem,* 2010, vol. 18 (5), 1925-1935
- **NEVES et al.** *Bioorg. Med. Chem.,* 2010, vol. 18 (5), 1925-1935
- **CHENG ; PRUSOFF.** *Biochem. Pharmacol.,* 1973, vol. 22, 3099-3108
- **KOJIMA.** *Prostate,* 2006, vol. 66, 761-767
- **LORKE.** *Arch Toxicol.,* 1983, vol. 54, 275-287
- **SOUZA ; DEAL.** *J Ethnopharmacol.,* 2011, vol. 135, 135-146